(19) Europäisches Patentamt
European Patent Office
Office européen des brevets

(11) Numéro de publication: **0 521 766 B1**

(12) **FASCICULE DE BREVET EUROPEEN**

(45) Date de publication de fascicule du brevet: **07.09.94**

(51) Int. Cl.5: **C07D 311/58**, C07D 405/12, C07B 57/00

(21) Numéro de dépôt: **92401846.8**

(22) Date de dépôt: **30.06.92**

(54) **Nouveau procédé de synthèse d'énantiomères de dérivés du 3-amino chromane.**

(30) Priorité: **01.07.91 FR 9108147**

(43) Date de publication de la demande:
**07.01.93 Bulletin 93/01**

(45) Mention de la délivrance du brevet:
**07.09.94 Bulletin 94/36**

(84) Etats contractants désignés:
**AT BE CH DE DK ES FR GB GR IT LI LU NL PT SE**

(56) Documents cités:
**EP-A- 0 222 996**
**EP-A- 0 452 204**
**US-A- 4 992 465**

(73) Titulaire: **ADIR ET COMPAGNIE**
**1 rue Carle Hébert**
**F-92415 Courbevoie Cédex (FR)**

(72) Inventeur: **Guillaumet, Gérald**
**2 rue Auguste Renoir**
**F-45100 Orléans (FR)**
Inventeur: **Fugier, Claude**
**34 rue André Caplet**
**F-76600 Le Havre (FR)**
Inventeur: **Souvie, Jean-Claude Justin**
**11 avenue Besselièvre**
**F-76210 Bolbec (FR)**
Inventeur: **Adam, Gérard**
**9, clos du Mesnil,**
**Route du Pecq**
**F-78600 Le Mesnil le Roi (FR)**
Inventeur: **Renard, Pierre**
**50 avenue de Villeneuve l'Etang**
**F-78000 Versailles (FR)**
Inventeur: **Caignard, Daniel-Henri**
**69 bis rue Brancion**
**F-75015 Paris (FR)**

EP 0 521 766 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un nouveau procédé de synthèse d'énantiomères de dérivés du 3-amino chromane.

Plus spécifiquement, la présente invention concerne un nouveau procédé de synthèse d'énantiomères de dérivés de formule générale (I) :

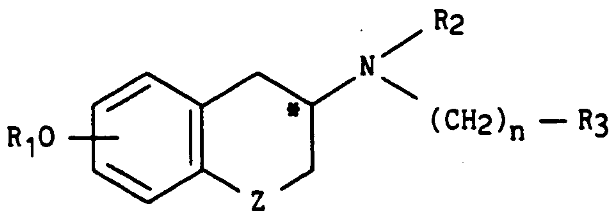

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement, alkyle ($C_1$-$C_6$) linéaire ou ramifié,
- n est un nombre entier compris entre 1 et 6,
- $R_3$ représente un groupement nitrile, un groupement amino éventuellement substitué par :
  - . un groupement acyle linéaire ou ramifié comprenant de 2 à 7 atome de carbone,
  - . un groupement alkylsulfonyl,
  - . un groupement arylsulfonyl éventuellement substitué par un groupement alkyle, alkoxy, hydroxy ou un atome d'halogène,
  - . un groupement alkyle ($C_1$-$C_6$) linéaire ou ramifié,

- ou $R_3$ représente l'un quelconque des groupements suivants :

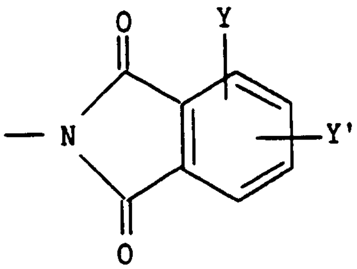 , 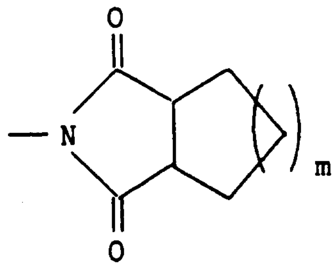 ,

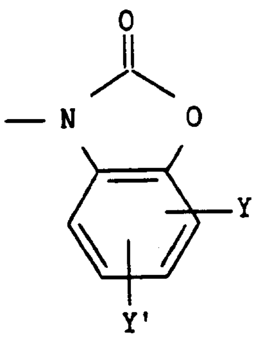 , 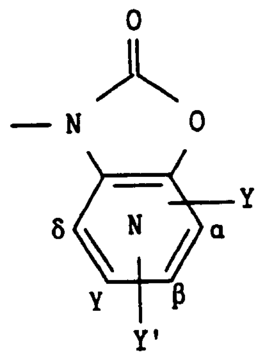 ,

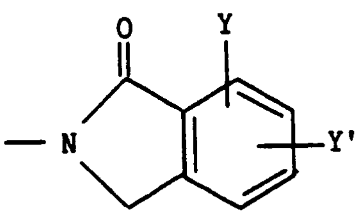 , 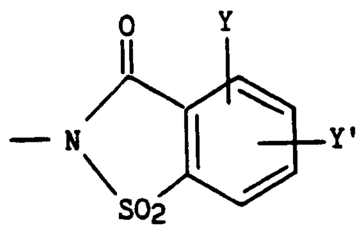 ,

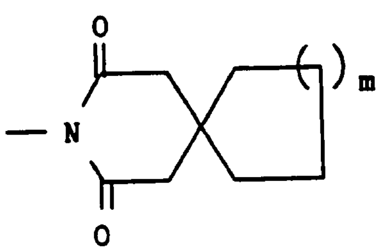 , 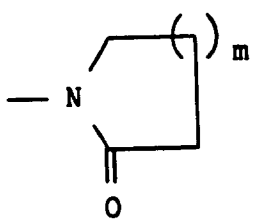 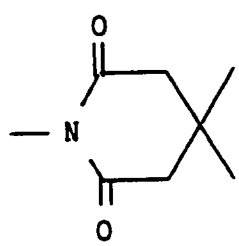 ,

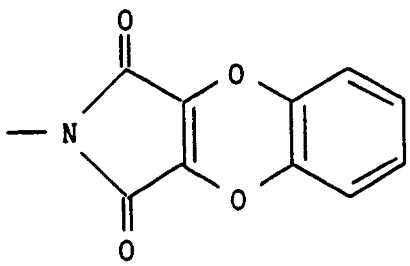

,

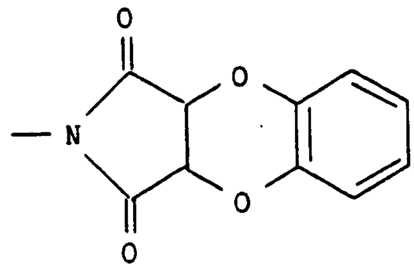

,

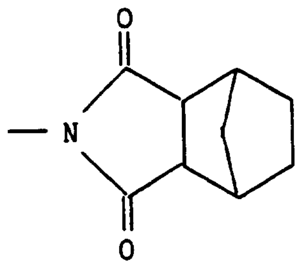

,

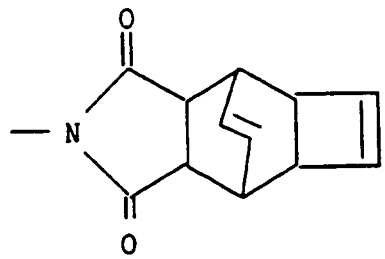

dans lesquels :

. Y et Y' identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position $\alpha$, $\beta$, $\gamma$ ou $\delta$ de la jonction du cycle,

leurs énantiomères, diastéréoisomères et épimères ainsi que leurs sels d'addition à un acide pharmaceutiquement acceptable.

Les composés de formule (I) ainsi que leurs sels d'addition à un acide pharmaceutiquement acceptable ont d'intéressantes propriétés pharmacologiques.

Les essais de binding ont montré que les composés de formule (I) se comportent comme de très puissants ligands des récepteurs 5-$HT_{1A}$, avec une activité agoniste ou antagoniste au niveau du système nerveux central. Cette très grande affinité est accompagnée d'une très grande sélectivité vis à vis de ces récepteurs par rapport aux récepteurs $D_2$ et $\alpha_2$.

Les composés de formule (I) trouvent donc leur application dans le traitement du stress, de l'anxiété, de la dépression, de la schizophrénie et de la douleur, des maladies cardiovasculaires et de l'hypertension. Ils peuvent également modifier le comportement alimentaire et sexuel.

Les composés de formule (I) présentent un carbone asymétrique porteur de la fonction azotée et existent donc sous forme de deux isomères qui selon la nomenclature introduite par R.S. Cahn, Sir C. Ingold et V. Prelog sont dénommés (R) et (S).

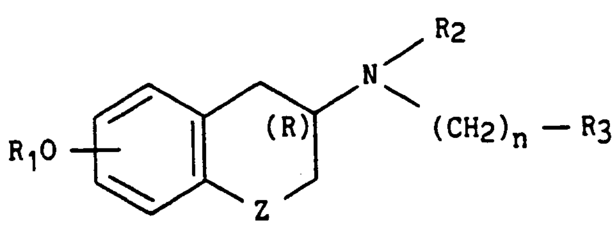

**(I/R)**

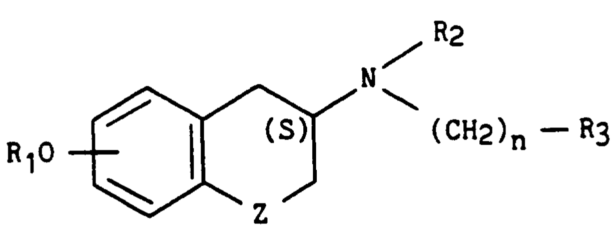

**(I/S)**

Les activités des composés de formule (I/R) et (I/S) sont assez différentes. La configuration du carbone asymétrique entraîne des modifications importantes de l'affinité et de la sélectivité du produit concerné envers les différentes catégories de récepteurs et partant sur son activité pharmacologique et ses effets secondaires. Par exemple le composé de formule (II) :

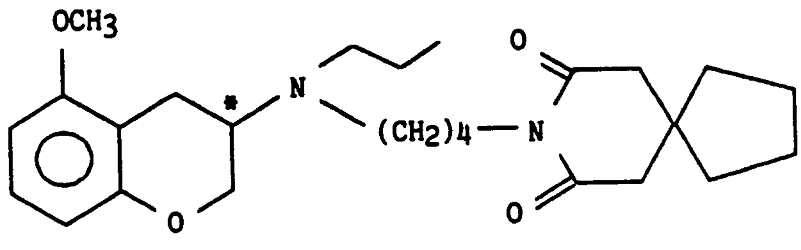

(II)

existe sous forme de deux énantiomères R et S dont le pouvoir rotatoire est évidemment différent. Ce pouvoir rotatoire est noté (+) ou (-) selon que l'énantiomère dévie la lumière polarisée vers la droite ou la gauche.

Si on utilise les techniques classiques de binding, l'affinité de l'isomère (+) du composé de formule (II) exprimé en K 0,5 est de $2 \times 10^{-10}$ pour les récepteurs $5HT_1A$ et de $2 \times 10^{-8}$ pour les récepteurs $D_2$ soit une sélectivité de l'ordre de 100.

En ce qui concerne l'isomère (-), le Ki est de 2 X 10-9 pour les récepteurs $5HT_1A$ et de $10^{-8}$ pour les récepteurs D2 soit une sélectivité de l'ordre de 5.

Ainsi, l'isomère (+) a une affinité pour les récepteurs $5HT_1A$ environ 10 fois supérieure à celle de l'isomère (-) et une spécificité 20 fois meilleure.

Il importe donc pour ces dérivés de pouvoir utiliser un procédé de synthèse permettant l'obtention sélective d'un de ces deux énantiomères.

Les procédés de synthèse des racémiques des composés de formule (I) ont été décrits dans la demande FR 90.04481 correspondant à la demande EP-A- 452 204, opposable seulement à la nouveauté de la présente invention.

Toutefois la demande EP-A-452 204 réalise la séparation des isomères au stade du 3-amino 2 chromane qui est la matière première et poursuit la synthèse à partir de l'énantiomère choisi. Or, la séparation est un étape délicate, onéreuse en produit et réaliser une synthèse à partir de matières premières qu'il convient de dédoubler au préalable n'est pas industriellement applicable car très onéreuse. A chaque étape de synthèse, les rendements n'étant jamais totalement quantitatifs, il y a perte de matière première et donc, perte financière.

La demanderesse a présentement découvert le moyen d'améliorer cette synthèse en ne réalisant la séparation des énantiomères qu'au stade des produits de formule (I), stade ultime de la synthèse. Le procédé de séparation des évantiomères de la présente invention est donc nouveau par rapport à la demande EP-A-452 204 puisqu'il décrit une séparation des énantiomères au stade final de la synthèse.

Pour cela, elle utilise une matière première de structure chromatique dépourvue de carbone asymétrique, une chromanone connue ou son homologue soufré, et de faible prix de revient.

Le procédé selon l'invention est plus particulièrement caractérisé en ce que l'on utilise comme matière première un dérivé de formule générale (III) :

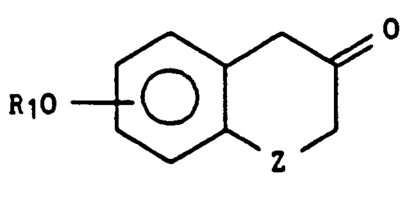

(III)

où $R_1$ et Z ont la même définition que dans la formule (I),
décrit dans la demande de brevet EP 0 222 996, que l'on soumet à amination réductive, préférentiellement à température et pression ambiantes, en présence d'un catalyseur, avec un dérivé de formule générale (IV) :

$NH_2 - (CH_2)_n - R_3$ (IV)

où n et $R_3$ ont la même définition que dans la formule (I),
pour obtenir un dérivé de formule (V) :

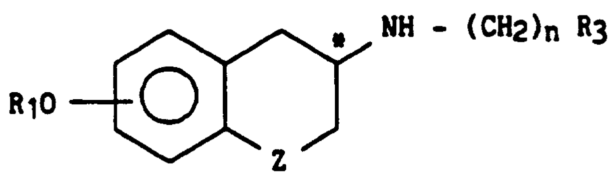

(V)

où $R_1$, $R_3$, Z et n ont la même définition que précédemment,
cas particulier des dérivés de formule (I) pour lesquels R2 représente un atome d'hydrogène,
dont on sépare les isomères (+) et (-) en en formant une solution organique et en y ajoutant un acide optiquement actif préférentiellement choisi parmi citrique, tartrique, diacétyl tartrique, maléique, malique, tosyl glutamique, camphosulfonique, camphorique, camphanique, mandélique, quinique, abandon à cristallisation, séparation des deux énantiomères, l'un précipitant, l'autre restant en suspension, ce qui permet leur séparation,
isomère choisi que l'on traite, lorsque dans le composé de formule (I) que l'on souhaite obtenir $R_2$ représente un groupement $C_1$-$C_6$ alkyle linéaire ou ramifié,
par un dérivé de formule (VI) :

$R'_2$ - X (VI)

dans laquelle X représente un atome d'halogène et $R'_2$ représente un groupement alkyle $C_1$-$C_6$ linéaire ou ramifié,
pour conduire spécifiquement à un énantiomère du dérivé de formule (I).

Une variante du procédé selon l'invention consiste lorsque dans le dérivé de formule (I) que l'on souhaite obtenir $R_2$ représente un groupement $C_1$-$C_6$ alkyle, à traiter le dérivé de formule (V) tel que précédemment défini par un dérivé de formule (VI) tel que précédemment défini, pour obtenir un dérivé de formule (I) sous forme de racémique dont on réalise la séparation des isomères (+) et (-) de façon analogue à celle décrite précédemment de façon générale pour les dérivés de formule (V).

Les sels d'acide optiquement actifs avec les dérivés de formule (I) sont nouveaux et font partie de l'invention en tant qu'intermédiaires utiles à sa réalisation.

Les exemples ci-dessous illustrent l'invention et ne la limitent en aucune façon.

La préparation ne fait pas partie de l'invention mais est utilisée pour la réalisation du procédé selon l'invention.

**PREPARATION : 5-METHOXY 3,4-DIHYDRO [2H] 1-BENZOPYRAN 3-ONE**

A 0,5 g (2,4 mmol) de 3-carboxy-5-méthoxy-2H-1-benzopyran obtenu comme décrit dans la demande PCT 88/04654 dans 5 ml de dichlorométhane, on ajoute 0,4 ml de triéthylamine puis goutte à goutte 0,65 g (2,4 mmol) de diphénylphosphoryl azide $[(C_6H_5O)_2\ P(O)N_3]$ en solution dans 2 ml de toluène. La solution est chauffée à 60 °C pour distiller le dichlorométhane et l'on ajoute 5 ml de toluène anhydre. Le mélange réactionnel est ensuite chauffé sous agitation à 80-85 °C pendant 1H30, puis on ajoute 4 ml d'une solution d'acide chlorhydrique 6N. Le mélange est à nouveau maintenu à reflux pendant 2 heures, puis refroidi à la température ambiante. Le produit est ensuite extrait plusieurs fois au dichlorométhane et le solvant d'extraction est séché sur $MgSO_4$ puis évaporé sous pression réduite.

La purification de l'échantillon brut obtenu, par chromatographie sur colonne de silice (éluant, $CH_2Cl_2$), permet d'avoir 0,246 g (57 %) de produit attendu sous forme d'huile qui devient cristallin par la suite à 4 °C (F = 53-54 °C).

La 5-méthoxy 3,4-dihydro 2H-1-benzopyran-3-one peut également être préparée comme indiquée dans la demande de brevet européen EP 0 222 996.

**EXEMPLE 1 : ⊕ 3-[4-[N-(5-METHOXY CHROMAN-3-YL)AMINO]BUTYL]2,4-DIOXO 3-AZASPIRO [4,5]-DECANE**

**STADE A : 3-[4-[N-(5-METHOXY CHROMAN-3-YL)AMINO]BUTYL]2,4-DIOXO 3-AZASPIRO [4,5]DECANE (CHLORHYDRATE)**

Dissoudre 0,04 mole de 5-méthoxy chroman-3-one dans 10 ml d'éthanol. Parallèlement dissoudre 1 g de chlorhydrate de N(4-amino butyl) 8-azaspiro [4,5]décane 7,9-dione dans 10 ml d'éthanol et ajouter à cette solution la solution de chromanone précédemment obtenue. Y ajouter ensuite en solution 0,16 g de soude dans 10 ml d'éthanol. Agiter pendant une heure à température ambiante, filtrer et placer dans un hydrogénateur. Ajouter 0,5 g de charbon palladié, purger à l'azote puis à l'hydrogène et hydrogéner à température et pression ambiantes. Filtrer et évaporer le filtrat à sec. Reprendre le résidu à l'acétate d'éthyle et y ajouter de l'acide chlorhydrique gazeux. Laisser précipiter et essorer.
Rendement : 70%

**STADE B : ⊕ 3-[4-[N-(5-METHOXY CHROMAN-3-YL)AMINO]BUTYL]2,4-DIOXO 3-AZASPIRO [4,5]-DECANE (CHLORHYDRATE)**

Dissoudre le produit obtenu au stade précédent dans 17 ml d'éthanol et ajouter 0,03 moles d'acide L(-) malique dissous dans 12,5 ml d'éthanol. Agiter pendant 30 minutes. Laisser à basse température.
Essorer le précipité formé, sécher.
Pouvoir rotatoire $\alpha_D^{21}$ (1%, $CHCl_3$) : + 15,3°

**EXEMPLE 2 : ⊕ 3-[4-[Nn PROPYL N-(5-METHOXY CHROMAN-3-YL)AMINO] BUTYL] 2,4-DIOXO 3-AZASPIRO [4,5]DECANE (OXALATE)**

1,5 mmoles du composé préparé dans l'exemple 1 est mis en solution dans 10 ml de diméthyl formamide en présence de 4,4 mmoles de 1-iodopropane et de 4,4 moles de carbonate de potassium. Après 24 heures d'agitation à 60°C le solvant est évaporé et le brut réactionnel est repris par 10 ml d'eau et extrait au dichlorométhane. La phase organique est séchée et évaporée et le produit attendu est obtenu après purification par chromatographie sur colonne de silice. Salifier par l'acide oxalique.
Rendement : 83%
Pouvoir rotatoire $\alpha_D^{21}$ : + 50°

**EXEMPLE 3 : ⊖ 3-[4-[N-(5-METHOXY CHROMAN-3-YL)AMINO] BUTYL] 2,4-DIOXO 3-AZASPIRO [4,5]-DECANE (CHLORHYDRATE)**

En procédant comme dans l'exemple 1, mais en remplaçant dans l'acide L(-) malique par l'acide D (+) malique, on obtient le produit du titre.

**EXEMPLE 4 : ⊖ 3-[4-[Nn PROPYL N-(5-METHOXY CHROMAN-3-YL)AMINO] BUTYL] 2,4-DIOXO 3-AZASPIRO [4,5]DECANE (OXALATE)**

En procédant comme dans l'exemple 2, mais en remplaçant le produit obtenu dans l'exemple 1 par le produit obtenu dans l'exemple 3, on obtient le produit du titre. Salifier par l'acide oxalique.
Pouvoir rotatoire $\alpha_D$ de l'amine libre $\alpha_D^{21}$ : -50 (4% $CHCl_3$)

**EXEMPLE 5 : ⊕ 3-[4-[N-(5-METHOXY CHROMAN-3-YL)AMINO] BUTYL] 2,4-DIOXO 3-AZASPIRO [4,5]-DECANE (CHLORHYDRATE)**

Dissoudre 2 g de produit obtenu dans l'exemple 1, stade A dans 3 cm3 d'éthanol. Ajouter à cette solution une solution de 1,08 g d'acide ⊖ camphanique dans 1,6 $cm^3$ d'éthanol. Agiter pendant 30 minutes. Laisser à basse température. Essorer le précipité formé. Sécher.

En procédant comme dans les exemples précédents sans salifier ni par l'acide chlorhydrique, ni par l'acide oxalique et en utilisant le dérivé de formule $NH_2-(CH_2)_n-R_3$ approprié on obtient de même :
**⊕ 5-METHOXY 3-[4-[Nn PROPYL N 2-(4-TOLUENE SULFONYLAMINO)ETHYL] AMINO] CHROMANE**
Pouvoir rotatoire $\alpha_D^{21}$ (4%, $CHCl_3$) : + 44°)
**⊖ 5-METHOXY 3-[Nn PROPYL N[2-(4-TOLUENE SULFONYLAMINO)ETHYL] AMINO] CHROMANE**

et les diastéréoisomères des composés suivants :

- **5-METHOXY 3-[Nn PROPYL N-[4-(4-TOLUENE SULFONYLAMINO)BUTYL] AMINO] CHROMANE**

Pouvoir rotatoire du diastéréoisomère ⊕ $\alpha_D^{21}$ (4%, $CHCl_3$) : +52°)

- **3-[4-[N-(5-METHOXY CHROMAN-3 YL)AMINO]BUTYL]2,4-DIOXO 3-AZABICYCLO [3.3.0] OCTANE**
- **3-[4-[Nn PROPYL N-(5-METHOXY CHROMAN-3 YL)AMINO]BUTYL]2,4-DIOXO 3-AZABICYCLO [3.3.0] OCTANE**

Pouvoir rotatoire du diastéréoisomère ⊕ $\alpha_D^{21}$ (4%, $CHCl_3$) : +57°)

- **3-[4-[N(5-METHOXY BENZOTHIOPYRAN-3-YL)AMINO]BUTYL]2,4-DIOXO 3-AZASPIRO [4,5] DECANE**
- **3-[4-[Nn PROPYL N(5-METHOXY BENZOTHIOPYRAN-3-YL)AMINO]BUTYL]2,4-DIOXO 3-AZABICYCLO [4,5] DECANE**
- **5-METHOXY 3-[Nn PROPYL N[2-(4-TOLUENE SULFONYLAMINO)ETHYL] AMINO]3,4-DIHYDRO [2H] 1- BENZOTHIOPYRANNE**
- **5-METHOXY 3-[Nn PROPYL N[4-(4-TOLUENE SULFONYLAMINO)BUTYL] AMINO]3,4-DIHYDRO [2H] BENZOTHIOPYRANNE**
- **5-METHOXY 3-[Nn PROPYL N[3-(4-TOLUENE SULFONYLAMINO)PROPYL] AMINO]3,4-DIHYDRO [2H] BENZOTHIOPYRANNE**

**Revendications**

**Revendications pour les Etats contractants suivants : AT, BE, CH, DE, DK, FR, GB, IT, LI, LU, NL, PT, SE**

**1.** Procédé de synthèse d'énantiomères de dérivés de formule générale (I) :

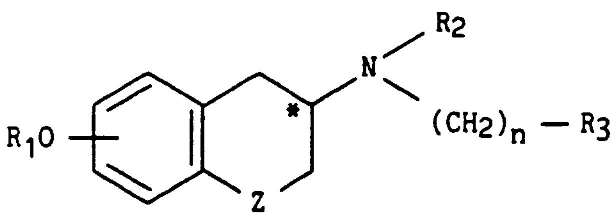

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  . un groupement acyle linéaire ou ramifié comprenant de 2 à 7 atome de carbone,
  . un groupement alkylsulfonyl,
  . un groupement arylsulfonyl éventuellement substitué par un groupement alkyle, alkoxy, hydroxy ou un atome d'halogène,
  . un groupement alkyle ($C_1$-$C_6$) linéaire ou ramifié,

- ou $R_3$ représente l'un quelconque des groupements suivants :

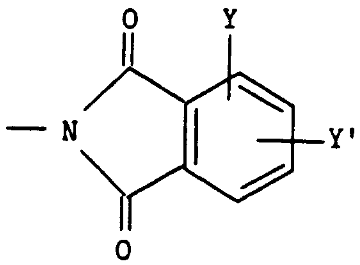 ,

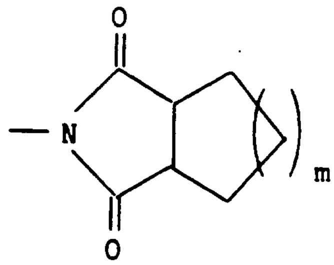 ,

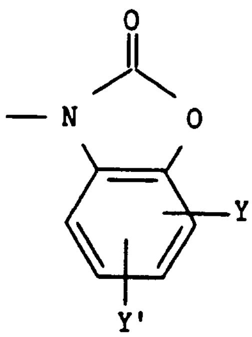 ,

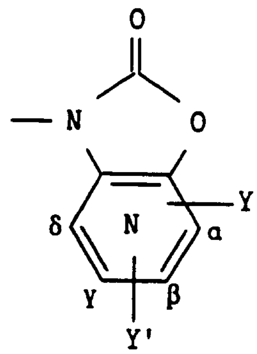 ,

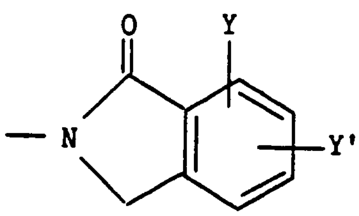 ,

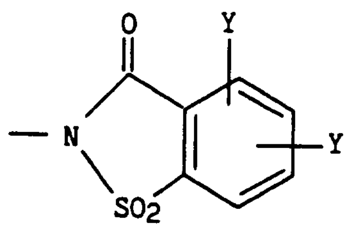 ,

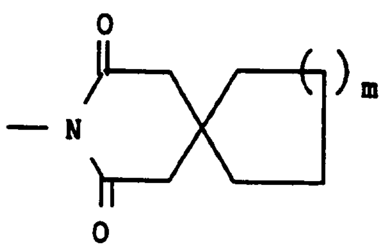 ,

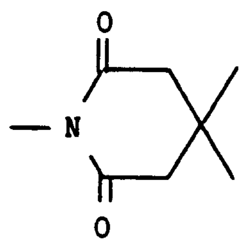 ,

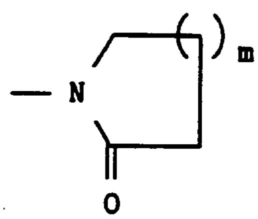

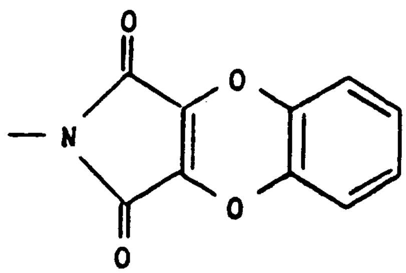 , 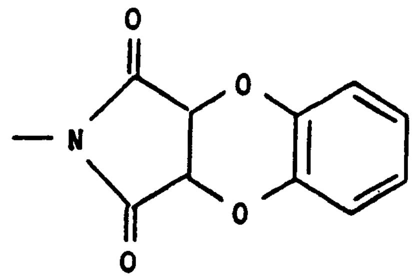 ,

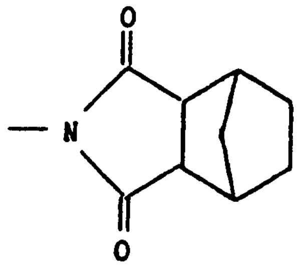 , 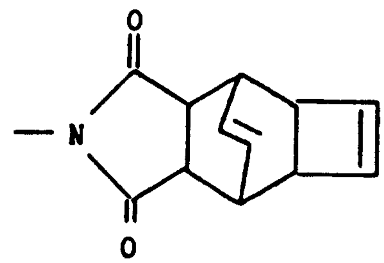

dans lesquels :

. Y et Y' identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position $\alpha$, $\beta$, $\gamma$ ou $\delta$ de la jonction du cycle,

leurs énantiomères, diastéréolsomères et épimères ainsi que leurs sels d'addition à un acide pharmaceutiquement acceptable,

caractérisé en ce que l'on utilise comme matière première un dérivé de formule (III) :

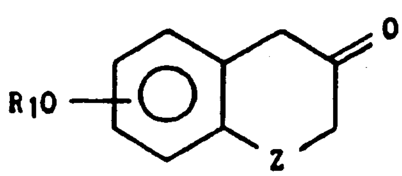

**(III)**

où $R_1$ et Z ont la même définition que dans la formule (I),

que l'on soumet à amination réductive préférentiellement à température et pression ambiantes en présence d'un catalyseur avec un dérivé de formule générale (IV) :

$NH_2 - (CH_2)_n - R_3$ (IV)

où n et $R_3$ ont la même définition que dans la formule (I),

pour obtenir un dérivé de formule (V) :

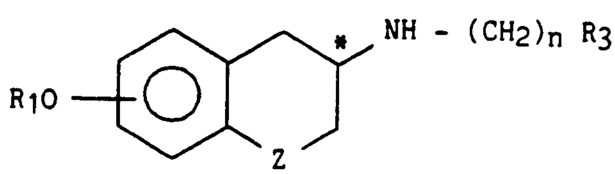

**(V)**

où $R_1$, $R_3$, Z et n ont la même définition que précédemment,

cas particulier des dérivés de formule (I) pour lesquels $R_2$ représente un atome d'hydrogène,

dont on sépare les isomères (+) et (-) en en formant une solution organique en y ajoutant un acide optiquement actif, et en abandonnant à cristallisation l'un des deux énantiomères cristallise, l'autre

restant en solution,
l'isomère choisi est alors traité, lorsque dans le composé de formule (I) que l'on souhaite obtenir $R_2$ représente un groupement $C_1$-$C_6$ alkyle linéaire ou ramifié,
par un dérivé de formule (VI) :

$R'_2$ - X (VI)

dans laquelle X représente un atome d'halogène et $R'_2$ représente un groupement alkyle $C_1$-$C_6$ linéaire ou ramifié,
pour conduire spécifiquement à un énantiomère du dérivé de formule (I).

**2.** Procédé de préparation d'énantiomères de dérivés de formule (I) selon la revendication 1 pour lesquels R2 ne représente pas un atome d'hydrogène, caractérisé en ce que l'on soumet à réaction un dérivé de formule (V) selon la revendication 1 avec un dérivé de formule (VI) selon la revendication 1, pour obtenir le racémique d'un composé de formule (I) selon la revendication 1 dont on sépare les énantiomères en en formant une solution organique en y ajoutant un acide optiquement actif et, en abandonnant à cristallisation l'un des deux énantiomères cristallisant, l'autre restant en solution.

**3.** Procédé de préparation d'énantiomères de composés de formule (I) selon la revendication 1 ou 2 caractérisé en ce que l'acide utilisé pour la séparation des énantiomères est choisi parmi (+) ou (-) citrique, tartrique, diacétyl tartrique, maléique, malique, tosyl glutamique, camphosulfonique, camphorique, camphanique, mandélique, quinique selon l'énantiomère du produit désiré.

**4.** Procédé de préparation d'énantiomères de dérivés de formule (I) selon la revendication 1 ou 2 caractérisé en ce que l'acide utilisé pour la séparation d'isomères est l'acide (+) ou (-) malique selon l'énantiomère désiré.

**5.** Procédé de préparation d'énantiomères de dérivés de formule (I) selon la revendication 1 ou 2 caractérisé en ce que l'acide utilisé pour la séparation des isomères est l'acide (+) ou (-) camphanique selon l'énantiomère désiré.

**6.** Procédé de préparation selon la revendication 1 ou 2 du dérivé de formule (I) qui est le ⊕ 3-[4-[N-(5-méthoxy chroman-3-yl)amino]butyl]2,4-dioxo 3-azaspiro [4,5]décane et ses sels d'addition à un acide pharmaceutiquement acceptable.

**7.** Procédé de préparation selon la revendication 1 ou 2 du dérivé de formule (I) qui est le ⊖ 3-[4-[n-(5-méthoxy chroman-3-yl)amino]butyl]2,4-dioxo 3-azaspiro [4,5]décane et ses sels d'addition à un acide pharmaceutiquement acceptable.

**8.** Procédé de préparation selon la revendication 1 ou 2 du dérivé de formule (I) qui est le ⊕ 3-[4-[Nn propyl N-(5-méthoxy chroman-3-yl)amino]butyl]2,4-dioxo 3-azaspiro [4,5]décane et ses sels d'addition à un acide pharmaceutiquement acceptable.

**9.** Procédé de préparation selon la revendication 1 ou 2 du dérivé de formule (I) qui est le ⊖ 3-[4-[Nn propyl N-(5-méthoxy chroman-3-yl)amino]butyl]2,4-dioxo 3-azaspiro [4,5]décane et ses sels d'addition à un acide pharmaceutiquement acceptable.

**10.** Procédé de préparation selon la revendication 1 ou 2 du composé de formule (I) qui est le ⊕ 5-méthoxy 3-[Nn propyl N-[4-(4-toluène sulfonylamino)butyl]amino]chromane] et ses sels d'addition à un acide pharmaceutiquement acceptable.

**11.** Procédé de préparation selon la revendication 1 ou 2 du composé de formule (I) qui est le ⊖ 5-méthoxy 3-[Nn propyl N-[4-(4-toluène sulfonylamino)butyl]amino]chromane] et ses sels d'addition à un acide pharmaceutiquement acceptable.

**12.** Procédé de préparation selon la revendication 1 ou 2 du composé de formule (I) qui est le ⊕ 5-méthoxy 3-[Nn propyl N-[2-(4-toluène sulfonylamino)éthyl]amino]chromane] et ses sels d'addition à un acide pharmaceutiquement acceptable.

**13.** Procédé de préparation selon la revendication 1 ou 2 du composé de formule (I) qui est le ⊖ 5-méthoxy 3-[Nn propyl N-[2-(4-toluène sulfonylamino)éthyl]amino]chromane] et ses sels d'addition à un acide pharmaceutiquement acceptable.

**14.** Procédé de préparation selon la revendication 1 ou 2 du composé de formule (I) qui est le ⊕ 3-{4-[Nn propyl N-(5-méthoxy chroman-3 yl)amino]butyl]2,4-dioxo} 3-azabicyclo [3,3,0] octane et ses sels d'addition à un acide pharmaceutiquement acceptable.

**15.** Procédé de préparation selon la revendication 1 ou 2 du composé de formule (I) qui est le ⊖ 3-{4-[Nn propyl N-(5-méthoxy chroman-3 yl)amino]butyl]2,4-dioxo} 3-azabicyclo [3,3,0] octane et ses sels d'addition à un acide pharmaceutiquement acceptable.

**16.** Sels d'addition de composés de formule (I) et de leurs énantiomères selon la revendication 1 avec les acides (+) ou (-) citrique, tartrique, diacétyl tartrique, maléique, malique, tosyl glutamique, camphosulfonique, camphorique, camphanique, mandélique, quinique.

**17.** Sels d'addition des composés de formule (I) et de leurs énantiomères selon la revendication 1 avec l'acide (+) ou (-) malique.

**18.** Sels d'addition des composés de formule (I) et de leurs énantiomères selons la revendication 1 avec l'acide (+) ou (-) camphanique.

**Revendications pour l'Etat contractant suivant : ES**

**1.** Procédé de synthèse d'énantiomères de dérivés de formule générale (I) :

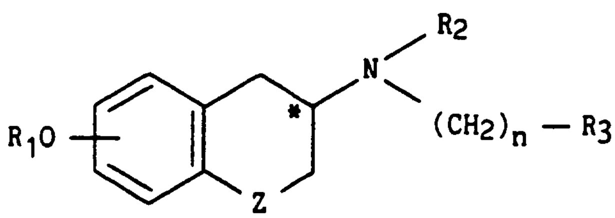

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  . un groupement acyle linéaire ou ramifié comprenant de 2 à 7 atome de carbone,
  . un groupement alkylsulfonyl,
  . un groupement arylsulfonyl éventuellement substitué par un groupement alkyle, alkoxy, hydroxy ou un atome d'halogène,
  . un groupement alkyle ($C_1$-$C_6$) linéaire ou ramifié,

- ou $R_3$ représente l'un quelconque des groupements suivants :

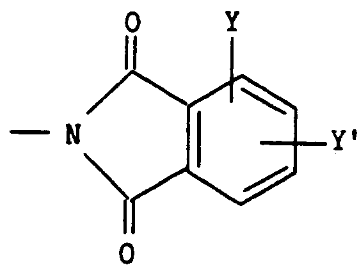

,

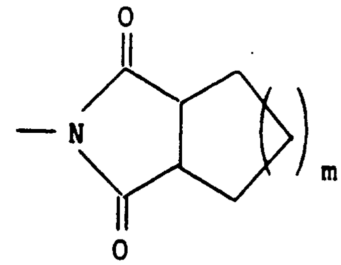

,

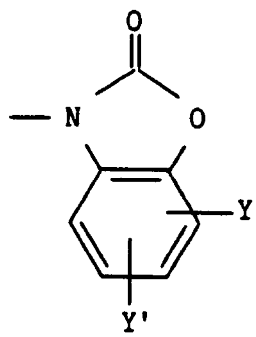

,

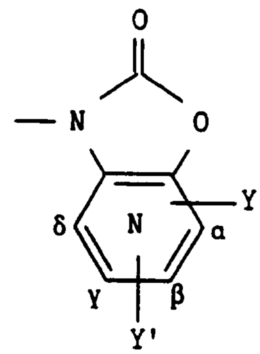

,

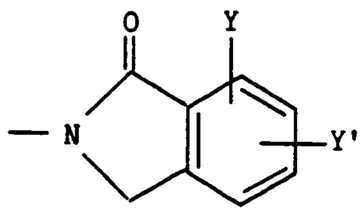

,

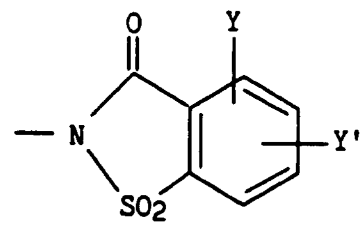

,

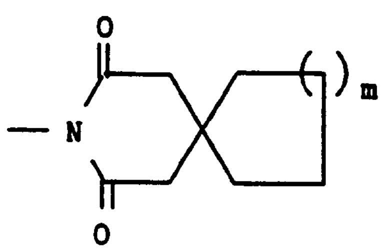

,

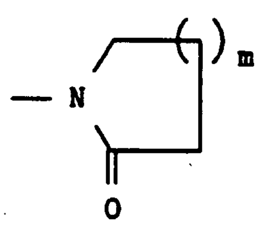

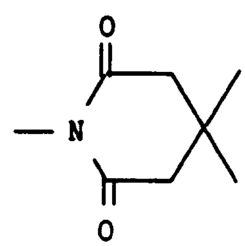

,

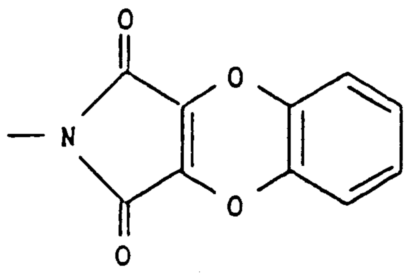
,
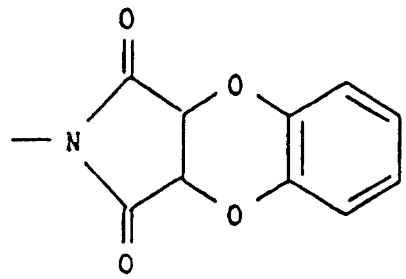
,
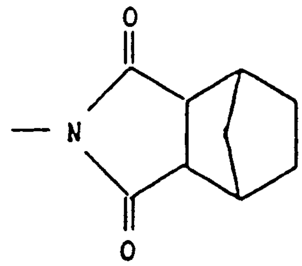
,
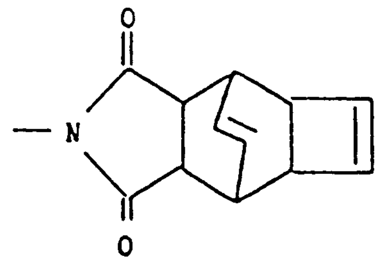

dans lesquels :

. Y et Y' identiques ou différents représentent un atome d'hydrogène, un atome d'halogène, un groupement alkyle, alkoxy ou hydroxy,
. m est un nombre entier égal à 1 ou 2,
. et l'azote du cycle pyridine se situe en position $\alpha$, $\beta$, $\gamma$ ou $\delta$ de la jonction du cycle,

leurs énantiomères, diastéréoisomères et épimères ainsi que leurs sels d'addition à un acide pharmaceutiquement acceptable,
caractérisé en ce que l'on utilise comme matière première un dérivé de formule (III) :

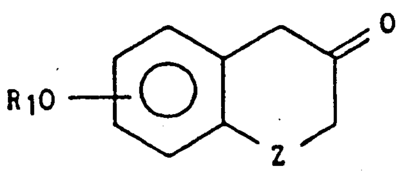
(III)

où $R_1$ et Z ont la même définition que dans la formule (I),
que l'on soumet à amination réductive préférentiellement à température et pression ambiantes en présence d'un catalyseur avec un dérivé de formule générale (IV) :

$NH_2 - (CH_2)_n - R_3$ (IV)

où n et $R_3$ ont la même définition que dans la formule (I),
pour obtenir un dérivé de formule (V) :

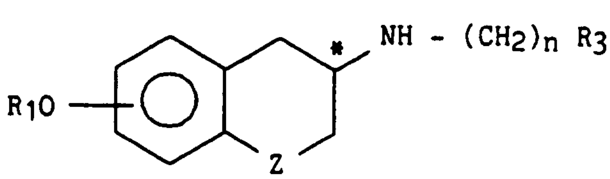
(V)

où $R_1$, $R_3$, Z et n ont la même définition que précédemment,
cas particulier des dérivés de formule (I) pour lesquels $R_2$ représente un atome d'hydrogène,
dont on sépare les isomères (+) et (-) en en formant une solution organique en y ajoutant un acide optiquement actif, et en abandonnant à cristallisation l'un des deux énantiomères cristallise, l'autre

restant en solution,
l'isomère choisi est alors traité, lorsque dans le composé de formule (I) que l'on souhaite obtenir $R_2$ représente un groupement $C_1$-$C_6$ alkyle linéaire ou ramifié,
par un dérivé de formule (VI) :

$R'_2$ - X (VI)

dans laquelle X représente un atome d'halogène et $R'_2$ représente un groupement alkyle $C_1$-$C_6$ linéaire ou ramifié,
pour conduire spécifiquement à un énantiomère du dérivé de formule (I).

**2.** Procédé de préparation d'énantiomères de dérivés de formule (I) selon la revendication 1 pour lesquels R2 ne représente pas un atome d'hydrogène, caractérisé en ce que l'on soumet à réaction un dérivé de formule (V) selon la revendication 1 avec un dérivé de formule (VI) selon la revendication 1, pour obtenir le racémique d'un composé de formule (I) selon la revendication 1 dont on sépare les énantiomères en en formant une solution organique en y ajoutant un acide optiquement actif et, en abandonnant à cristallisation l'un des deux énantiomères cristallisant, l'autre restant en solution.

**3.** Procédé de préparation d'énantiomères de composés de formule (I) selon la revendication 1 ou 2 caractérisé en ce que l'acide utilisé pour la séparation des énantiomères est choisi parmi (+) ou (-) citrique, tartrique, diacétyl tartrique, maléique, malique, tosyl glutamique, camphosulfonique, camphorique, camphanique, mandélique, quinique selon l'énantiomère du produit désiré.

**4.** Procédé de préparation d'énantiomères de dérivés de formule (I) selon la revendication 1 ou 2 caractérisé en ce que l'acide utilisé pour la séparation d'isomères est l'acide (+) ou (-) malique selon l'énantiomère désiré.

**5.** Procédé de préparation d'énantiomères de dérivés de formule (I) selon la revendication 1 ou 2 caractérisé en ce que l'acide utilisé pour la séparation des isomères est l'acide (+) ou (-) camphanique selon l'énantiomère désiré.

**6.** Procédé de préparation selon la revendication 1 ou 2 du dérivé de formule (I) qui est le ⊕ 3-[4-[N-(5-méthoxy chroman-3-yl)amino]butyl]2,4-dioxo 3-azaspiro [4,5]décane et ses sels d'addition à un acide pharmaceutiquement acceptable.

**7.** Procédé de préparation selon la revendication 1 ou 2 du dérivé de formule (I) qui est le ⊖ 3-[4-[n-(5-méthoxy chroman-3-yl)amino]butyl]2,4-dioxo 3-azaspiro [4,5]décane et ses sels d'addition à un acide pharmaceutiquement acceptable.

**8.** Procédé de préparation selon la revendication 1 ou 2 du dérivé de formule (I) qui est le ⊕ 3-[4-[Nn propyl N-(5-méthoxy chroman-3-yl)amino]butyl]2,4-dioxo 3-azaspiro [4,5]décane et ses sels d'addition à un acide pharmaceutiquement acceptable.

**9.** Procédé de préparation selon la revendication 1 ou 2 du dérivé de formule (I) qui est le ⊖ 3-[4-[Nn propyl N-(5-méthoxy chroman-3-yl)amino]butyl]2,4-dioxo 3-azaspiro [4,5]decane et ses sels d'addition à un acide pharmaceutiquement acceptable.

**10.** Procédé de préparation selon la revendication 1 ou 2 du composé de formule (I) qui est le ⊕ 5-méthoxy 3-[Nn propyl N-[4-(4-toluène sulfonylamino)butyl]amino]chromane] et ses sels d'addition à un acide pharmaceutiquement acceptable.

**11.** Procédé de préparation selon la revendication 1 ou 2 du composé de formule (I) qui est le ⊖ 5-méthoxy 3-[Nn propyl N-[4-(4-toluène sulfonylamino)butyl]amino]chromane] et ses sels d'addition à un acide pharmaceutiquement acceptable.

**12.** Procédé de préparation selon la revendication 1 ou 2 du composé de formule (I) qui est le ⊕ 5-méthoxy 3-[Nn propyl N-[2-(4-toluène sulfonylamino)éthyl]amino]chromane] et ses sels d'addition à un acide pharmaceutiquement acceptable.

**13.** Procédé de préparation selon la revendication 1 ou 2 du composé de formule (I) qui est le ⊖ 5-méthoxy 3-[Nn propyl N-[2-(4-toluène sulfonylamino)éthyl]amino]chromane] et ses sels d'addition à un acide pharmaceutiquement acceptable.

**14.** Procédé de préparation selon la revendication 1 ou 2 du composé de formule (I) qui est le ⊕ 3-{4-[Nn propyl N-(5-méthoxy chroman-3 yl)amino]butyl]2,4-dioxo} 3-azabicyclo [3,3,0] octane et ses sels d'addition à un acide pharmaceutiquement acceptable.

**15.** Procédé de préparation selon la revendication 1 ou 2 du composé de formule (I) qui est le ⊖ 3-{4-[Nn propyl N-(5-méthoxy chroman-3 yl)amino]butyl]2,4-dioxo} 3-azabicyclo [3,3,0] octane et ses sels d'addition à un acide pharmaceutiquement acceptable.

**16.** Sels d'addition de composés de formule (I) et de leurs énantiomères selon la revendication 1 avec les acides (+) ou (-) citrique, tartrique, diacétyl tartrique, maléique, malique, tosyl glutamique, camphosulfonique, camphorique, camphanique, mandélique, quinique.

**17.** Sels d'addition des composés de formule (I) et de leurs énantiomères selon la revendication 1 avec l'acide (+) ou (-) malique.

**18.** Sels d'addition des composés de formule (I) et de leurs énantiomères selons la revendication 1 avec l'acide (+) ou (-) camphanique.

**Revendications pour l'Etat contractant suivant : GR**

**1.** Procédé de synthèse d'énantiomères de dérivés de formule générale (I) :

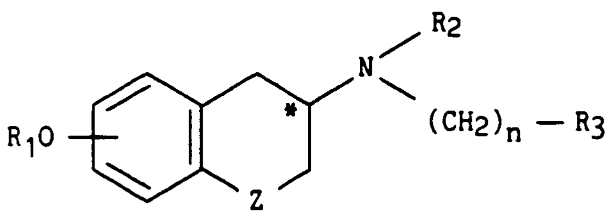

(I)

dans laquelle :

- Z représente un atome d'oxygène ou un atome de soufre,
- $R_1$ représente un atome d'hydrogène, ou un groupement alkyle ($C_1$-$C_6$) linéaire ou ramifié,
- $R_2$ représente un atome d'hydrogène ou un groupement alkyle ($C_1$-$C_6$) linéaire ou ramifié,
- n est un nombre entier compris entre 1 et 6,
- $R_3$ représente un groupement nitrile, un groupement amino éventuellement substitué par :
  - . un groupement acyle linéaire ou ramifié comprenant de 2 à 7 atome de carbone,
  - . un groupement alkylsulfonyl,
  - . un groupement arylsulfonyl éventuellement substitué par un groupement alkyle, alkoxy, hydroxy ou un atome d'halogène,
  - . un groupement alkyle ($C_1$-$C_6$) linéaire ou ramifié,

- ou $R_3$ représente l'un quelconque des groupements suivants :

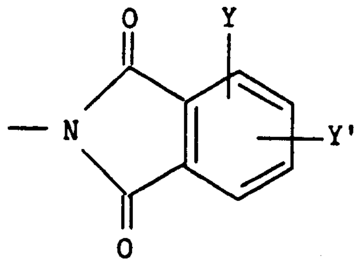

,

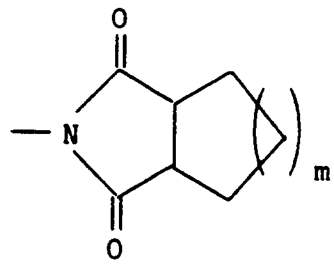

,

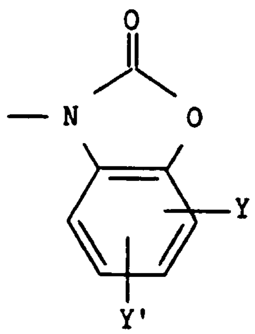

,

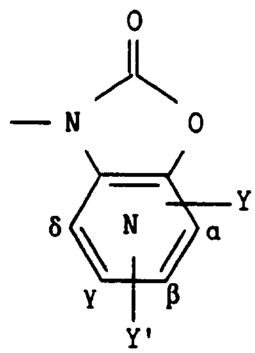

,

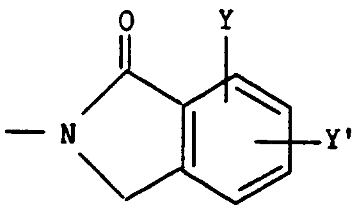

,

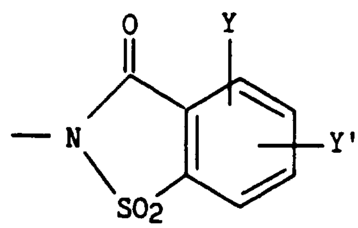

,

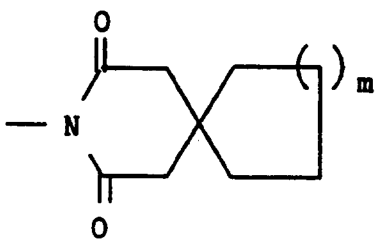

,

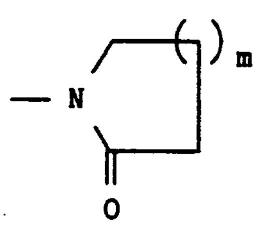

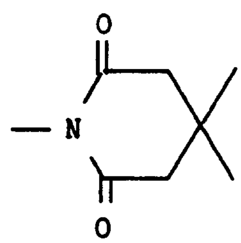

,

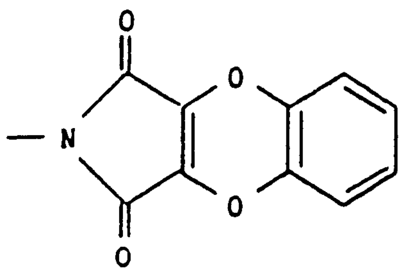 , 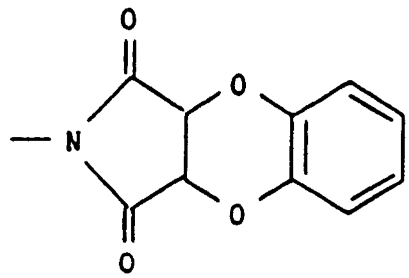 ,

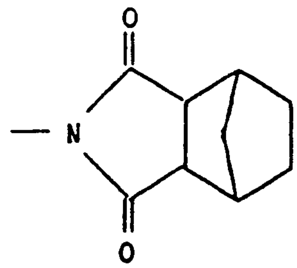 , 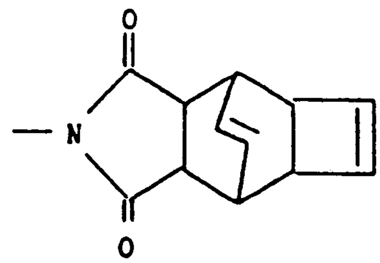

dans lesquels :

. Y et Y' identiques ou différents représentent un atome d'hydrogène, un atome d'halogène, un groupement alkyle, alkoxy ou hydroxy,

. m est un nombre entier égal à 1 ou 2,

. et l'azote du cycle pyridine se situe en position $\alpha$, $\beta$, $\gamma$ ou $\delta$ de la jonction du cycle,

leurs énantiomères, diastéréoisomères et épimères ainsi que leurs sels d'addition à un acide pharmaceutiquement acceptable,

caractérisé en ce que l'on utilise comme matière première un dérivé de formule (III) :

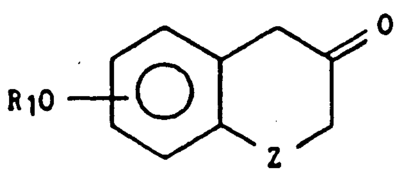 (III)

où $R_1$ et Z ont la même définition que dans la formule (I),

que l'on soumet à amination réductive préférentiellement à température et pression ambiantes en présence d'un catalyseur avec un dérivé de formule générale (IV) :

$NH_2 - (CH_2)_n - R_3$ (IV)

où n et $R_3$ ont le même définition que dans la formule (I),

pour obtenir un dérivé de formule (V) :

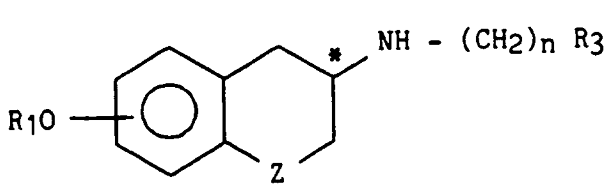 (V)

où $R_1$, $R_3$, Z et n ont la même définition que précédemment,

cas particulier des dérivés de formule (I) pour lesquels $R_2$ représente un atome d'hydrogène,

dont on sépare les isomères (+) et (-) en en formant une solution organique en y ajoutant un acide optiquement actif, et en abandonnant à cristallisation l'un des deux énantiomères cristallise, l'autre

restant en solution,
l'isomère choisi est alors traité, lorsque dans le composé de formule (I) que l'on souhaite obtenir $R_2$ représente un groupement $C_1$-$C_6$ alkyle linéaire ou ramifié,
par un dérivé de formule (VI) :

$R'_2$ - X (VI)

dans laquelle X représente un atome d'halogène et $R'_2$ représente un groupement alkyle $C_1$-$C_6$ linéaire ou ramifié,
pour conduire spécifiquement à un énantiomère du dérivé de formule (I).

**2.** Procédé de préparation d'énantiomères de dérivés de formule (I) selon la revendication 1 pour lesquels R2 ne représente pas un atome d'hydrogène, caractérisé en ce que l'on soumet à réaction un dérivé de formule (V) selon la revendication 1 avec un dérivé de formule (VI) selon la revendication 1, pour obtenir le racémique d'un composé de formule (I) selon la revendication 1 dont on sépare les énantiomères en en formant une solution organique en y ajoutant un acide optiquement actif et, en abandonnant à cristallisation l'un des deux énantiomères cristallisant, l'autre restant en solution.

**3.** Procédé de préparation d'énantiomères de composés de formule (I) selon la revendication 1 ou 2 caractérisé en ce que l'acide utilisé pour la séparation des énantiomères est choisi parmi (+) ou (-) citrique, tartrique, diacétyl tartrique, maléique, malique, tosyl glutamique, camphosulfonique, camphorique, camphanique, mandélique, quinique selon l'énantiomère du produit désiré.

**4.** Procédé de préparation d'énantiomères de dérivés de formule (I) selon la revendication 1 ou 2 caractérisé en ce que l'acide utilisé pour la séparation d'isomères est l'acide (+) ou (-) malique selon l'énantiomère désiré.

**5.** Procédé de préparation d'énantiomères de dérivés de formule (I) selon la revendication 1 ou 2 caractérisé en ce que l'acide utilisé pour la séparation des isomères est l'acide (+) ou (-) camphanique selon l'énantiomère désiré.

**6.** Procédé de préparation selon la revendication 1 ou 2 du dérivé de formule (I) qui est le ⊕ 3-[4-[N-(5-méthoxy chroman-3-yl)amino]butyl]2,4-dioxo 3-azaspiro [4,5]décane et ses sels d'addition à un acide pharmaceutiquement acceptable.

**7.** Procédé de préparation selon la revendication 1 ou 2 du dérivé de formule (I) qui est le ⊖ 3-[4-[n-(5-méthoxy chroman-3-yl)amino]butyl]2,4-dioxo 3-azaspiro [4,5]décane et ses sels d'addition à un acide pharmaceutiquement acceptable.

**8.** Procédé de préparation selon la revendication 1 ou 2 du dérivé de formule (I) qui est le ⊕ 3-[4-[Nn propyl N-(5-méthoxy chroman-3-yl)amino]butyl]2,4-dioxo 3-azaspiro [4,5]décane et ses sels d'addition à un acide pharmaceutiquement acceptable.

**9.** Procédé de préparation selon la revendication 1 ou 2 du dérivé de formule (I) qui est le ⊖ 3-[4-[Nn propyl N-(5-méthoxy chroman-3-yl)amino]butyl]2,4-dioxo 3-azaspiro [4,5]décane et ses sels d'addition à un acide pharmaceutiquement acceptable.

**10.** Procédé de préparation selon la revendication 1 ou 2 du composé de formule (I) qui est le ⊕ 5-méthoxy 3-[Nn propyl N-[4-(4-toluène sulfonylamino)butyl]amino]chromane] et ses sels d'addition à un acide pharmaceutiquement acceptable.

**11.** Procédé de préparation selon la revendication 1 ou 2 du composé de formule (I) qui est le ⊖ 5-méthoxy 3-[Nn propyl N-[4-(4-toluène sulfonylamino)butyl]amino]chromane] et ses sels d'addition à un acide pharmaceutiquement acceptable.

**12.** Procédé de préparation selon la revendication 1 ou 2 du composé de formule (I) qui est le ⊕ 5-méthoxy 3-[Nn propyl N-[2-(4-toluène sulfonylamino)éthyl]amino]chromane] et ses sels d'addition à un acide pharmaceutiquement acceptable.

**13.** Procédé de préparation selon la revendication 1 ou 2 du composé de formule (I) qui est le ⊖ 5-méthoxy 3-[Nn propyl N-[2-(4-toluène sulfonylamino)éthyl]amino]chromane] et ses sels d'addition à un acide pharmaceutiquement acceptable.

**14.** Procédé de préparation selon la revendication 1 ou 2 du composé de formule (I) qui est le ⊕ 3-{4-[Nn propyl N-(5-méthoxy chroman-3 yl)amino]butyl]2,4-diozo} 3-azabicyclo [3,3,0] octane et ses sels d'addition à un acide pharmaceutiquement acceptable.

**15.** Procédé de préparation selon la revendication 1 ou 2 du composé de formule (I) qui est le ⊖ 3-{4-[Nn propyl N-(5-méthoxy chroman-3 yl)amino]butyl]2,4-dioxo} 3-azabicyclo [3,3,0] octane et ses sels d'addition à un acide pharmaceutiquement acceptable.

**16.** Sels d'addition de composés de formule (I) et de leurs énantiomères selon la revendication 1 avec les acides (+) ou (-) citrique, tartrique, diacétyl tartrique, maléique, malique, tosyl glutamique, camphosulfonique, camphorique, camphanique, mandélique, quinique.

**17.** Sels d'addition des composés de formule (I) et de leurs énantiomères selon la revendication 1 avec l'acide (+) ou (-) malique.

**18.** Sels d'addition des composés de formule (I) et de leurs énantiomères selons la revendication 1 avec l'acide (+) ou (-) camphanique.

**Claims**

**Claims for the following Contracting States : AT, BE, CH, DE, DK, FR, GB, IT, LI, LU, NL, PT, SE**

**1.** Process for the synthesis of enantiomers of compounds of the general formula (I):

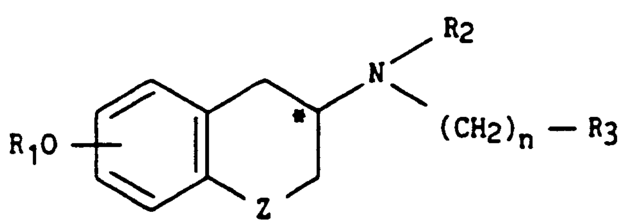

(I)

wherein:

- Z represents an oxygen atom or a sulphur atom,
- $R_1$ represents a hydrogen atom or a linear or branched ($C_1$-$C_6$)-alkyl grouping,
- $R_2$ represents a hydrogen atom or a linear or branched ($C_1$-$C_6$)-alkyl grouping,
- n represents an integer from 1 to 6,
- $R_3$ represents a nitrile grouping, an amino grouping optionally substituted by:
  . a linear or branched acyl grouping containing from 2 to 7 carbon atoms,
  . an alkylsulphonyl grouping,
  . an arylsulphonyl grouping optionally substituted by an alkyl, alkoxy or hydroxy grouping or a halogen atom,
  . a linear or branched ($C_1$-$C_6$)-alkyl grouping,

- or $R_3$ represents any one of the following groupings:

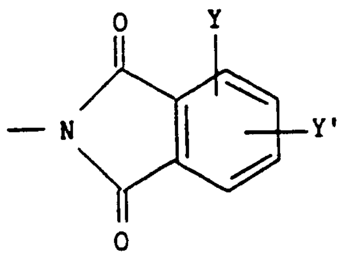 , 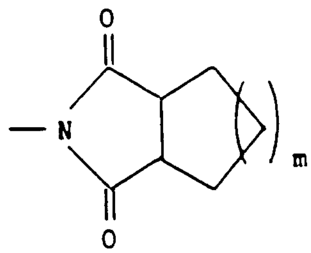 ,

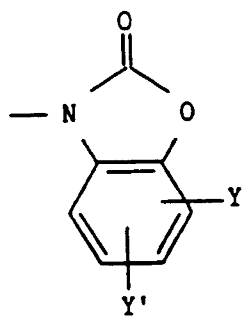 , 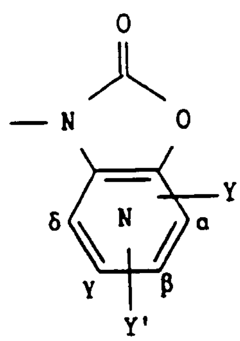 ,

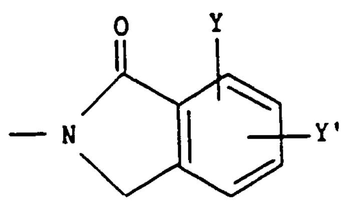 , 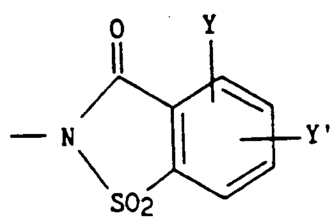 ,

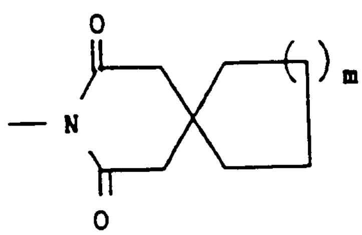 , 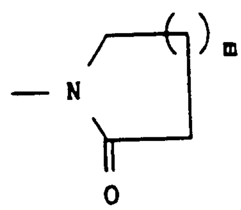 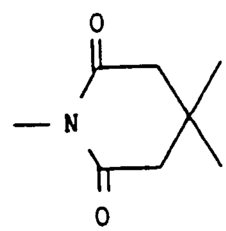 ,

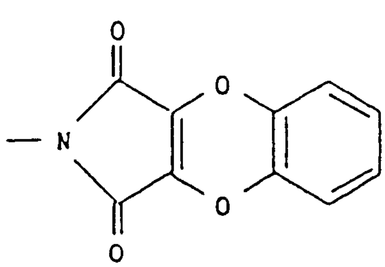 , 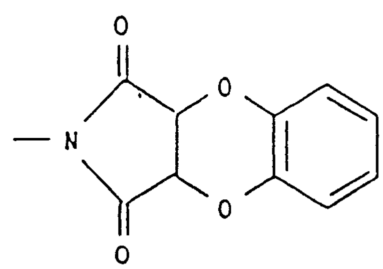 ,

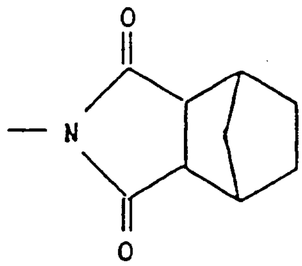

,

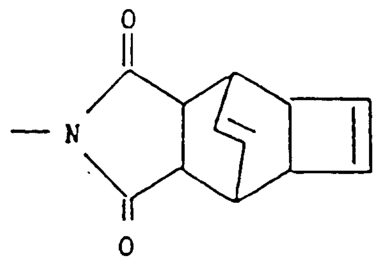

wherein:

- . Y and Y', which may be identical or different, represent a hydrogen atom, a halogen atom, or an alkyl, alkoxy or hydroxy grouping,
- . m is the integer 1 or 2,
- . and the nitrogen of the pyridine ring is in the $\alpha$-, $\beta$-, $\gamma$- or $\delta$-ring junction position,

their enantiomers, diastereoisomers and epimers and also their addition salts with a pharmaceutically acceptable acid,
characterised in that there is used as starting material a compound of formula (III):

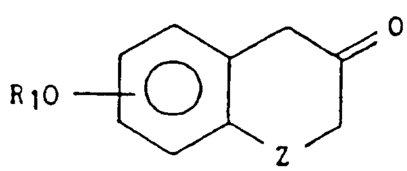

(III)

wherein $R_1$ and Z are as defined in formula (I),
which is subjected to reductive amination, preferably at ambient temperature and pressure in the presence of a catalyst, with a compound of the general formula (IV):

$NH_2 - (CH_2)_n - R_3$ (IV)

wherein n and $R_3$ are as defined in formula (I),
to obtain a compound of formula (V):

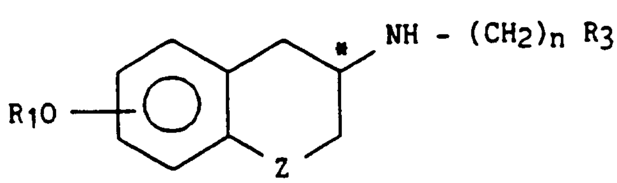

(V)

wherein $R_1$, $R_3$, Z and n are as defined above,
a particular case of the compounds of formula (I) wherein $R_2$ represents a hydrogen atom,
the (+)- and (-)-isomers of which are separated by forming therefrom an organic solution by adding thereto an optically active acid, and, on being left to crystallise, one of the two enantiomers crystallises while the other remains in solution,
the chosen isomer is then treated, when in the compound of formula (I) that it is desired to obtain $R_2$ represents a linear or branched $C_1$-$C_6$-alkyl grouping,
with a compound of formula (VI):

$R'_2 - X$ (VI)

wherein X represents a halogen atom and $R'_2$ represents a linear or branched $C_1$-$C_6$-alkyl grouping, to yield specifically an enantiomer of the compound of formula (I).

2. Process for the preparation of enantiomers of compounds of formula (I) according to claim 1 wherein $R_2$ does not represent a hydrogen atom, characterised in that a compound of formula (V) according to claim 1 is reacted with a compound of formula (VI) according to claim 1, to obtain the racemate of a compound of formula (I) according to claim 1, the enantiomers of which are separated by forming therefrom an organic solution by adding thereto an optically active acid, and, on being left to crystallise, one of the two enantiomers crystallises while the other remains in solution.

3. Process for the preparation of enantiomers of compounds of formula (I) according to claim 1 or 2, characterised in that the acid used for the separation of the enantiomers is selected from (+)- or (-)-citric, -tartaric, -diacetyltartaric, -maleic, -malic, -tosyl-glutamic, -camphosulphonic, -camphoric, -camphanic, -mandelic and -quinic acid, depending on which enantiomer of the product is desired.

4. Process for the preparation of enantiomers of compounds of formula (I) according to claim 1 or 2, characterised in that the acid used for the separation of isomers is (+)- or (-)-malic acid, depending on which enantiomer is desired.

5. Process for the preparation of enantiomers of compounds of formula (I) according to claim 1 or 2, characterised in that the acid used for the separation of the isomers is (+)- or (-)-camphanic acid, depending on which enantiomer is desired.

6. Process for the preparation according to claim 1 or 2 of the compound of formula (I) that is ⊕3-[4-[N-(5-methoxychroman-3-yl)amino]butyl]-2,4-dioxo-3-azaspiro-[4.5]decane and its addition salts with a pharmaceutically acceptable acid.

7. Process for the preparation according to claim 1 or 2 of the compound of formula (I) that is ⊖3-[4-[N-(5-methoxychroman-3-yl)amino]butyl]-2,4-dioxo-3-azaspiro-[4.5]decane and its addition salts with a pharmaceutically acceptable acid.

8. Process for the preparation according to claim 1 or 2 of the compound of formula (I) that is ⊕3-[4-[N-n-propyl-N-(5-methoxychroman-3-yl)amino]butyl]-2,4-dioxo-3-azaspiro[4.5]decane and its addition salts with a pharmaceutically acceptable acid.

9. Process for the preparation according to claim 1 or 2 of the compound of formula (I) that is ⊖3-[4-[N-n-propyl-N-(5-methoxychroman-3-yl)amino]butyl]-2,4-dioxo-3-azaspiro[4.5]decane and its addition salts with a pharmaceutically acceptable acid.

10. Process for the preparation according to claim 1 or 2 of the compound of formula (I) that is ⊕5-methoxy-3-[N-n-propyl-N-[4-(4-toluenesulphonylamino)butyl]amino]-chroman and its addition salts with a pharmaceutically acceptable acid.

11. Process for the preparation according to claim 1 or 2 of the compound of formula (I) that is ⊖5-methoxy-3-[N-n-propyl-N-[4-(4-toluenesulphonylamino)butyl]amino]-chroman and its addition salts with a pharmaceutically acceptable acid.

12. Process for the preparation according to claim 1 or 2 of the compound of formula (I) that is ⊕5-methoxy-3-[N-n-propyl-N-[2-(4-toluenesulphonylamino)ethyl]amino]-chroman and its addition salts with a pharmaceutically acceptable acid.

13. Process for the preparation according to claim 1 or 2 of the compound of formula (I) that is ⊖5-methoxy-3-[N-n-propyl-N-[2-(4-toluenesulphonylamino)ethyl]amino]chroman and its addition salts with a pharmaceutically acceptable acid.

14. Process for the preparation according to claim 1 or 2 of the compound of formula (I) that is ⊕3-{4-[N-n-propyl-N-(5-methoxychroman-3-yl)amino]butyl}-2,4-dioxo-3-azabicyclo[3.3.0]octane and its addition

salts with a pharmaceutically acceptable acid.

**15.** Process for the preparation according to claim 1 or 2 of the compound of formula (I) that is ⊖3-{4-[N-n-propyl-N-(5-methoxychroman-3-yl)amino]butyl}-2,4-dioxo-3-azabicyclo[3.3.0]octane and its addition salts with a pharmaceutically acceptable acid.

**16.** Addition salts of compounds of formula (I) and their enantiomers according to claim 1 with (+)- or (-)-citric, -tartaric, -diacetyltartaric, -maleic, -malic, -tosyl-glutamic, -camphosulphonic, -camphoric, -camphanic, -mandelic or -quinic acid.

**17.** Addition salts of the compounds of formula (I) and of their enantiomers according to claim 1 with (+)- or (-)-malic acid.

**18.** Addition salts of the compounds of formula (I) and of their enantiomers according to claim 1 with (+)- or (-)-camphanic acid.

**Claims for the following Contracting State : ES**

**1.** Process for the synthesis of enantiomers of compounds of the general formula (I):

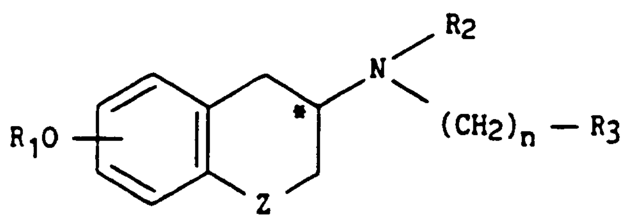

(I)

wherein:

- Z represents an oxygen atom or a sulphur atom,
- $R_1$ represents a hydrogen atom or a linear or branched ($C_1$-$C_6$)-alkyl grouping,
- $R_2$ represents a hydrogen atom or a linear or branched ($C_1$-$C_6$)-alkyl grouping,
- n represents an integer from 1 to 6,
- $R_3$ represents a nitrile grouping, an amino grouping optionally substituted by:
  - . a linear or branched acyl grouping containing from 2 to 7 carbon atoms,
  - . an alkylsulphonyl grouping,
  - . an arylsulphonyl grouping optionally substituted by an alkyl, alkoxy or hydroxy grouping or a halogen atom,
  - . a linear or branched ($C_1$-$C_6$)-alkyl grouping,

- or $R_3$ represents any one of the following groupings:

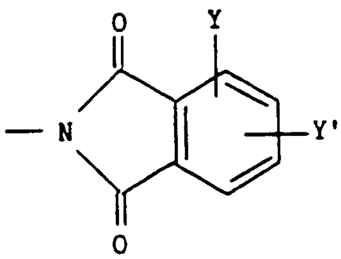 , 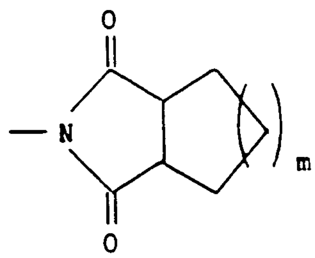 ,

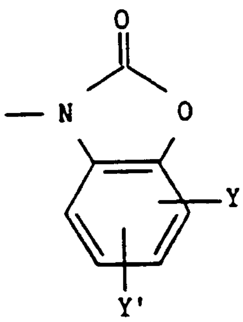 , 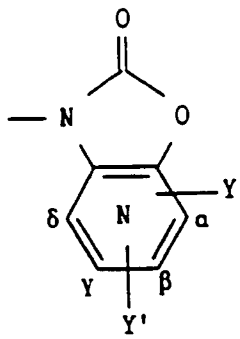 ,

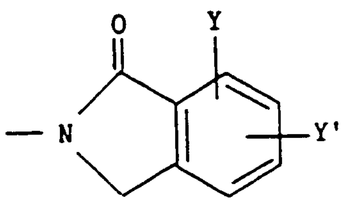 , 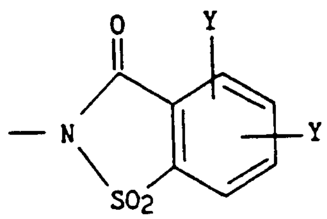 ,

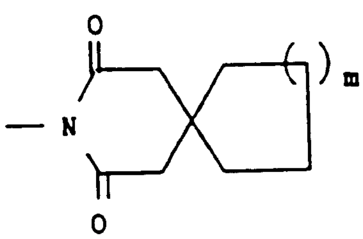 , 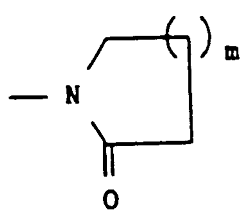 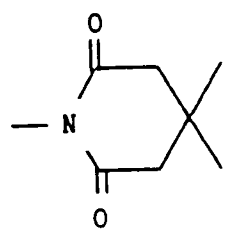 ,

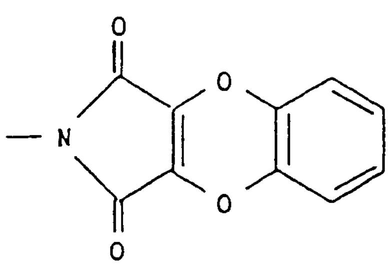 , 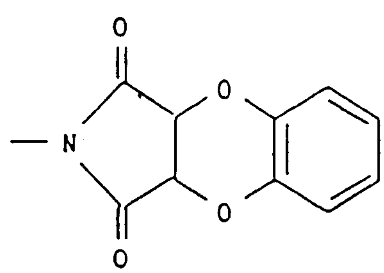 ,

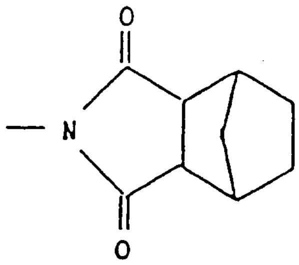

,

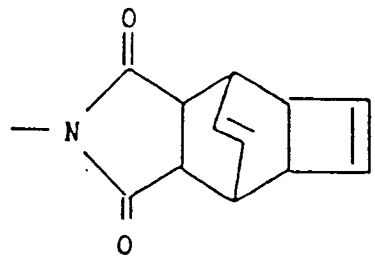

wherein:

- Y and Y', which may be identical or different, represent a hydrogen atom, a halogen atom, or an alkyl, alkoxy or hydroxy grouping,
- m is the integer 1 or 2,
- and the nitrogen of the pyridine ring is in the α-, β-, γ- or δ-ring junction position,

their enantiomers, diastereoisomers and epimers and also their addition salts with a pharmaceutically acceptable acid,
characterised in that there is used as starting material a compound of formula (III):

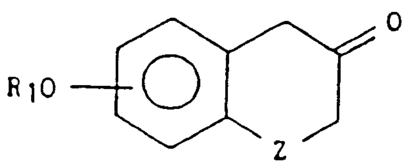

(III)

wherein $R_1$ and Z are as defined in formula (I),
which is subjected to reductive amination, preferably at ambient temperature and pressure in the presence of a catalyst, with a compound of the general formula (IV):

$NH_2$ - $(CH_2)_n$- $R_3$ (IV)

wherein n and $R_3$ are as defined in formula (I),
to obtain a compound of formula (V):

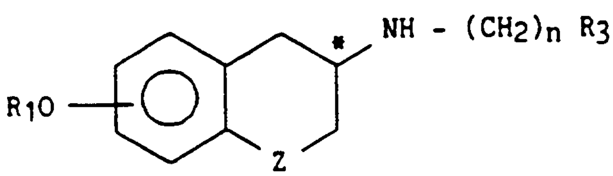

(V)

wherein $R_1$, $R_3$, Z and n are as defined above,
a particular case of the compounds of formula (I) wherein $R_2$ represents a hydrogen atom,
the (+)- and (-)-isomers of which are separated by forming therefrom an organic solution by adding thereto an optically active acid, and, on being left to crystallise, one of the two enantiomers crystallises while the other remains in solution,
the chosen isomer is then treated, when in the compound of formula (I) that it is desired to obtain $R_2$ represents a linear or branched $C_1$-$C_6$-alkyl grouping,
with a compound of formula (VI):

$R'_2$ - X (VI)

wherein X represents a halogen atom and $R'_2$ represents a linear or branched $C_1$-$C_6$-alkyl grouping, to yield specifically an enantiomer of the compound of formula (I).

**2.** Process for the preparation of enantiomers of compounds of formula (I) according to claim 1 wherein $R_2$ does not represent a hydrogen atom, characterised in that a compound of formula (V) according to claim 1 is reacted with a compound of formula (VI) according to claim 1, to obtain the racemate of a compound of formula (I) according to claim 1, the enantiomers of which are separated by forming therefrom an organic solution by adding thereto an optically active acid, and, on being left to crystallise, one of the two enantiomers crystallises while the other remains in solution.

**3.** Process for the preparation of enantiomers of compounds of formula (I) according to claim 1 or 2, characterised in that the acid used for the separation of the enantiomers is selected from (+)- or (-)-citric, -tartaric, -diacetyltartaric, -maleic, -malic, -tosyl-glutamic, -camphosulphonic, -camphoric, -camphanic, -mandelic and -quinic acid, depending on which enantiomer of the product is desired.

**4.** Process for the preparation of enantiomers of compounds of formula (I) according to claim 1 or 2, characterised in that the acid used for the separation of isomers is (+)- or (-)-malic acid, depending on which enantiomer is desired.

**5.** Process for the preparation of enantiomers of compounds of formula (I) according to claim 1 or 2, characterised in that the acid used for the separation of the isomers is (+)- or (-)-camphanic acid, depending on which enantiomer is desired.

**6.** Process for the preparation according to claim 1 or 2 of the compound of formula (I) that is ⊕3-[4-[N-(5-methoxychroman-3-yl)amino]butyl]-2,4-dioxo-3-azaspiro-[4.5]decane and its addition salts with a pharmaceutically acceptable acid.

**7.** Process for the preparation according to claim 1 or 2 of the compound of formula (I) that is ⊖3-[4-[N-(5-methoxychroman-3-yl)amino]butyl]-2,4-dioxo-3-azaspiro-[4.5]decane and its addition salts with a pharmaceutically acceptable acid.

**8.** Process for the preparation according to claim 1 or 2 of the compound of formula (I) that is ⊕3-[4-[N-n-propyl-N-(5-methoxychroman-3-yl)amino]butyl]-2,4-dioxo-3-azaspiro[4.5]decane and its addition salts with a pharmaceutically acceptable acid.

**9.** Process for the preparation according to claim 1 or 2 of the compound of formula (I) that is ⊖3-[4-[N-n-propyl-N-(5-methoxychroman-3-yl)amino]butyl]-2,4-dioxo-3-azaspiro[4.5]decane and its addition salts with a pharmaceutically acceptable acid.

**10.** Process for the preparation according to claim 1 or 2 of the compound of formula (I) that is ⊕5-methoxy-3-[N-n-propyl-N-[4-(4-toluenesulphonylamino)butyl]amino]chroman and its addition salts with a pharmaceutically acceptable acid.

**11.** Process for the preparation according to claim 1 or 2 of the compound of formula (I) that is ⊖5-methoxy-3-[N-n-propyl-N-[4-(4-toluenesulphonylamino)butyl]amino]chroman and its addition salts with a pharmaceutically acceptable acid.

**12.** Process for the preparation according to claim 1 or 2 of the compound of formula (I) that is ⊕5-methoxy-3-[N-n-propyl-N-[2-(4-toluenesulphonylamino)ethyl]amino]chroman and its addition salts with a pharmaceutically acceptable acid.

**13.** Process for the preparation according to claim 1 or 2 of the compound of formula (I) that is ⊖5-methoxy-3-[N-n-propyl-N-[2-(4-toluenesulphonylamino)ethyl]amino]chroman and its addition salts with a pharmaceutically acceptable acid.

**14.** Process for the preparation according to claim 1 or 2 of the compound of formula (I) that is ⊕3-{4-[N-n-propyl-N-(5-methoxychroman-3-yl)amino]butyl}-2,4-dioxo-3-azabicyclo[3.3.0]octane and its addition salts with a pharmaceutically acceptable acid.

**15.** Process for the preparation according to claim 1 or 2 of the compound of formula (I) that is ⊖3-{4-[N-n-propyl-N-(5-methoxychroman-3-yl)amino]butyl}-2,4-dioxo-3-azabicyclo[3.3.0]octane and its addition salts with a pharmaceutically acceptable acid.

**16.** Addition salts of compounds of formula (I) and their enantiomers according to claim 1 with (+)- or (-)-citric, -tartaric, -diacetyltartaric, -maleic, -malic, -tosyl-glutamic, -camphosulphonic, -camphoric, -camphanic, -mandelic or -quinic acid.

**17.** Addition salts of the compounds of formula (I) and of their enantiomers according to claim 1 with (+)- or (-)-malic acid.

**18.** Addition salts of the compounds of formula (I) and of their enantiomers according to claim 1 with (+)- or (-)-camphanic acid.

**Claims for the following Contracting State : GR**

**1.** Process for the synthesis of enantiomers of compounds of the general formula (I):

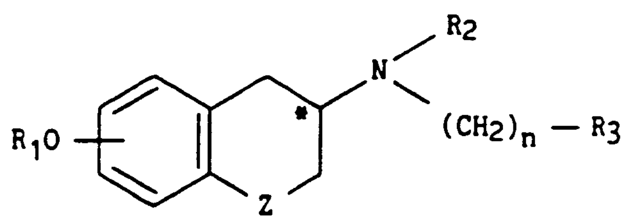

(I)

wherein:

- Z represents an oxygen atom or a sulphur atom,
- $R_1$ represents a hydrogen atom or a linear or branched ($C_1$-$C_6$)-alkyl grouping,
- $R_2$ represents a hydrogen atom or a linear or branched ($C_1$-$C_6$)-alkyl grouping,
- n represents an integer from 1 to 6,
- $R_3$ represents a nitrile grouping, an amino grouping optionally substituted by:
  - . a linear or branched acyl grouping containing from 2 to 7 carbon atoms,
  - . an alkylsulphonyl grouping,
  - . an arylsulphonyl grouping optionally substituted by an alkyl, alkoxy or hydroxy grouping or a halogen atom,
  - . a linear or branched ($C_1$-$C_6$)-alkyl grouping,

- or $R_3$ represents any one of the following groupings:

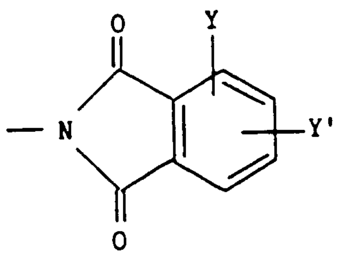 , 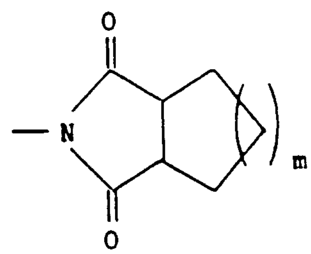 ,

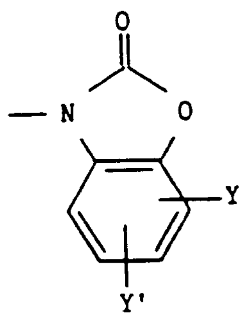 , 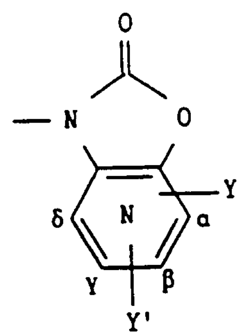 ,

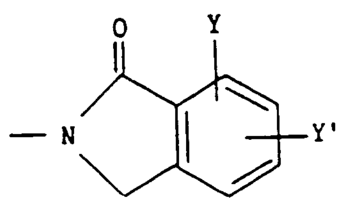 , 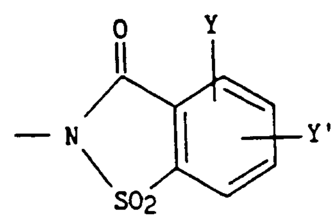 ,

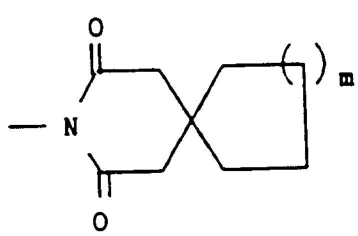 , 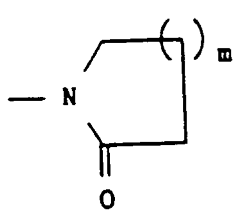 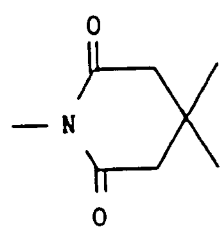 ,

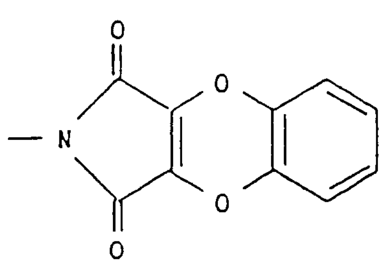 , 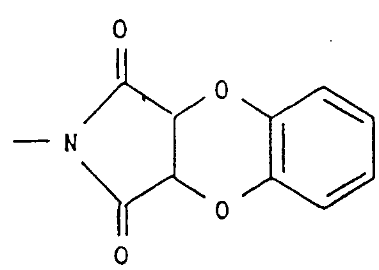 ,

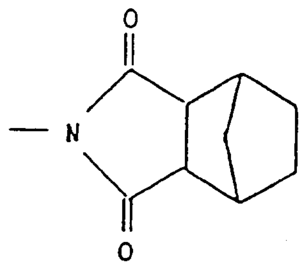 , 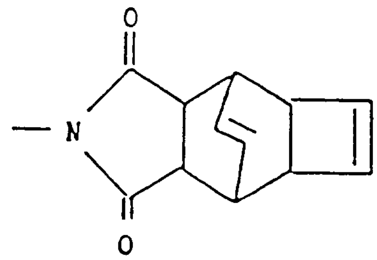

wherein:

- . Y and Y', which may be identical or different, represent a hydrogen atom, a halogen atom, or an alkyl, alkoxy or hydroxy grouping,
- . m is the integer 1 or 2,
- . and the nitrogen of the pyridine ring is in the $\alpha$-, $\beta$-, $\gamma$- or $\delta$-ring junction position,

their enantiomers, diastereoisomers and epimers and also their addition salts with a pharmaceutically acceptable acid,
characterised in that there is used as starting material a compound of formula (III):

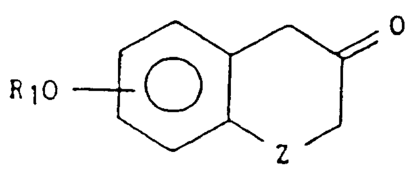

(III)

wherein $R_1$ and Z are as defined in formula (I),
which is subjected to reductive amination, preferably at ambient temperature and pressure in the presence of a catalyst, with a compound of the general formula (IV):

$NH_2$ - $(CH_2)_n$- $R_3$ (IV)

wherein n and $R_3$ are as defined in formula (I),
to obtain a compound of formula (V):

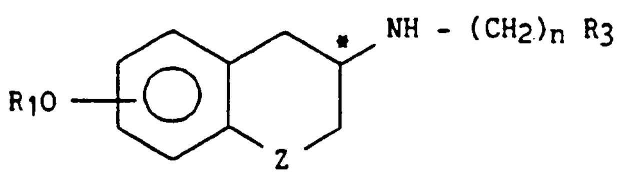

(V)

wherein $R_1$, $R_3$, Z and n are as defined above,
a particular case of the compounds of formula (I) wherein $R_2$ represents a hydrogen atom,
the (+)- and (-)-isomers of which are separated by forming therefrom an organic solution by adding thereto an optically active acid, and, on being left to crystallise, one of the two enantiomers crystallises while the other remains in solution,
the chosen isomer is then treated, when in the compound of formula (I) that it is desired to obtain $R_2$ represents a linear or branched $C_1$-$C_6$-alkyl grouping,
with a compound of formula (VI):

$R'_2$ - X (VI)

wherein X represents a halogen atom and $R'_2$ represents a linear or branched $C_1$-$C_6$-alkyl grouping,
to yield specifically an enantiomer of the compound of formula (I).

2. Process for the preparation of enantiomers of compounds of formula (I) according to claim 1 wherein $R_2$ does not represent a hydrogen atom, characterised in that a compound of formula (V) according to claim 1 is reacted with a compound of formula (VI) according to claim 1, to obtain the racemate of a compound of formula (I) according to claim 1, the enantiomers of which are separated by forming therefrom an organic solution by adding thereto an optically active acid, and, on being left to crystallise, one of the two enantiomers crystallises while the other remains in solution.

3. Process for the preparation of enantiomers of compounds of formula (I) according to claim 1 or 2, characterised in that the acid used for the separation of the enantiomers is selected from (+)- or (-)-citric, -tartaric, -diacetyltartaric, -maleic, -malic, -tosyl-glutamic, -camphosulphonic, -camphoric, -camphanic, -mandelic and -quinic acid, depending on which enantiomer of the product is desired.

4. Process for the preparation of enantiomers of compounds of formula (I) according to claim 1 or 2, characterised in that the acid used for the separation of isomers is (+)- or (-)-malic acid, depending on which enantiomer is desired.

5. Process for the preparation of enantiomers of compounds of formula (I) according to claim 1 or 2, characterised in that the acid used for the separation of the isomers is (+)- or (-)-camphanic acid, depending on which enantiomer is desired.

6. Process for the preparation according to claim 1 or 2 of the compound of formula (I) that is ⊕3-[4-[N-(5-methoxychroman-3-yl)amino]butyl]-2,4-dioxo-3-azaspiro-[4.5]decane and its addition salts with a pharmaceutically acceptable acid.

7. Process for the preparation according to claim 1 or 2 of the compound of formula (I) that is ⊖3-[4-[N-(5-methoxychroman-3-yl)amino]butyl]-2,4-dioxo-3-azaspiro-[4.5]decane and its addition salts with a pharmaceutically acceptable acid.

8. Process for the preparation according to claim 1 or 2 of the compound of formula (I) that is ⊕3-[4-[N-n-propyl-N-(5-methoxychroman-3-yl)amino]butyl]-2,4-dioxo-3-azaspiro[4.5]decane and its addition salts with a pharmaceutically acceptable acid.

9. Process for the preparation according to claim 1 or 2 of the compound of formula (I) that is ⊖3-[4-[N-n-propyl-N-(5-methoxychroman-3-yl)amino]butyl]-2,4-dioxo-3-azaspiro[4.5]decane and its addition salts with a pharmaceutically acceptable acid.

10. Process for the preparation according to claim 1 or 2 of the compound of formula (I) that is ⊕5-methoxy-3-[N-n-propyl-N-[4-(4-toluenesulphonylamino)butyl]amino]chroman and its addition salts with a pharmaceutically acceptable acid.

11. Process for the preparation according to claim 1 or 2 of the compound of formula (I) that is ⊖5-methoxy-3-[N-n-propyl-N-[4-(4-toluenesulphonylamino)butyl]amino]chroman and its addition salts with a pharmaceutically acceptable acid.

12. Process for the preparation according to claim 1 or 2 of the compound of formula (I) that is ⊕5-methoxy-3-[N-n-propyl-N-[2-(4-toluenesulphonylamino)ethyl]amino]chroman and its addition salts with a pharmaceutically acceptable acid.

13. Process for the preparation according to claim 1 or 2 of the compound of formula (I) that is ⊖5-methoxy-3-[N-n-propyl-N-[2-(4-toluenesulphonylamino)ethyl]amino]chroman and its addition salts with a pharmaceutically acceptable acid.

14. Process for the preparation according to claim 1 or 2 of the compound of formula (I) that is ⊕3-{4-[N-n-propyl-N-(5-methoxychroman-3-yl)amino]butyl}-2,4-dioxo-3-azabicyclo[3.3.0]octane and its addition salts with a pharmaceutically acceptable acid.

15. Process for the preparation according to claim 1 or 2 of the compound of formula (I) that is ⊖3-{4-[N-n-propyl-N-(5-methoxychroman-3-yl)amino]butyl}-2,4-dioxo-3-azabicyclo[3.3.0]octane and its addition

salts with a pharmaceutically acceptable acid.

**16.** Addition salts of compounds of formula (I) and their enantiomers according to claim 1 with (+)- or (-)-citric, -tartaric, -diacetyltartaric, -maleic, -malic, -tosyl-glutamic, -camphosulphonic, -camphoric, -camphanic, -mandelic or -quinic acid.

**17.** Addition salts of the compounds of formula (I) and of their enantiomers according to claim 1 with (+)- or (-)-malic acid.

**18.** Addition salts of the compounds of formula (I) and of their enantiomers according to claim 1 with (+)- or (-)-camphanic acid.

**Patentansprüche**
**Patentansprüche für folgende Vertragsstaaten : AT, BE, CH, DE, DK, FR, GB, IT, LI, LU, NL, PT, SE**

**1.** Verfahren zur Herstellung von Enantiomeren von Derivaten der allgemeinen Formel (I):

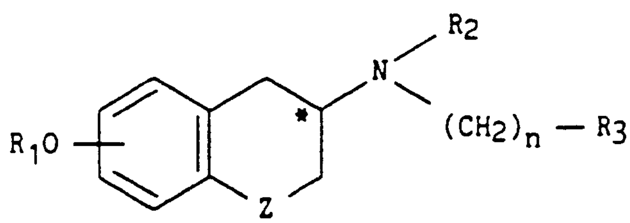

(I)

in der

- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl mit einem Wert von 1 bis 6,
- $R_3$ eine Nitrilgruppe, eine Aminogruppe, die gegebenenfalls durch eine
  . geradkettige oder verzweigte Acylgruppe mit 2 bis 7 Kohlenstoffatomen,
  . eine Alkylsulfonylgruppe,
  . eine Arylsulfonylgruppe, die gegebenenfalls durch eine Alkylgruppe, eine Alkoxygruppe, eine Hydroxylgruppe oder ein Halogenatom substituiert ist, oder
  . eine geradkettige oder verzweigte ($C_1$-$C_6$)-Alkylgruppe substituiert ist,

- oder $R_3$ eine der folgenden Gruppen:

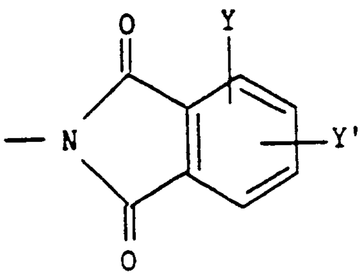

,

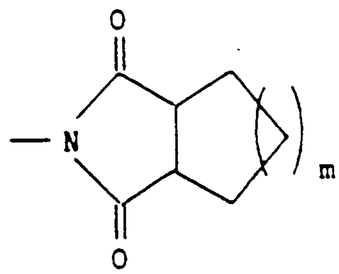

,

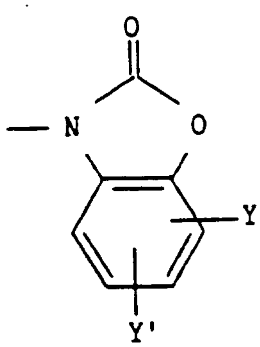

,

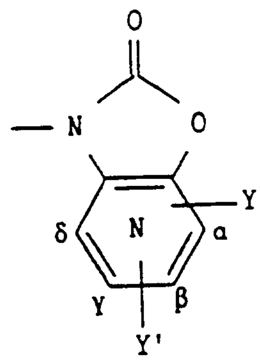

,

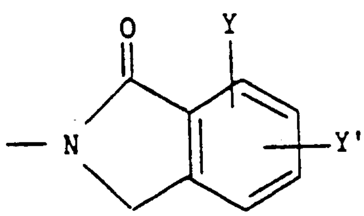

,

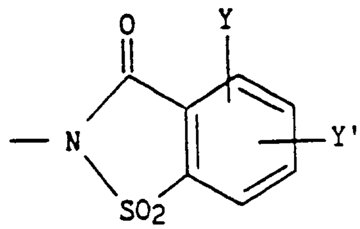

,

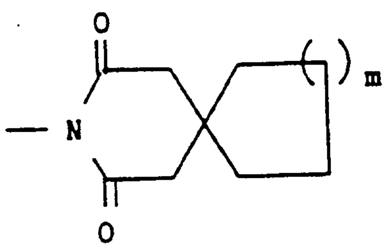

,

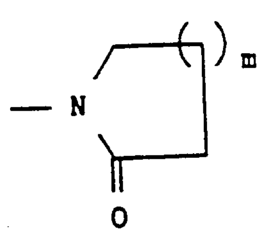

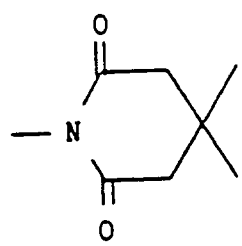

,

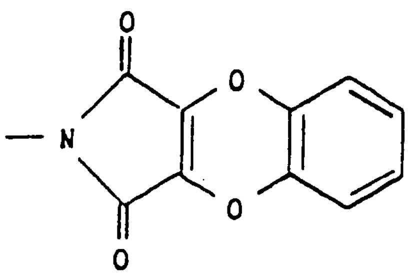
,
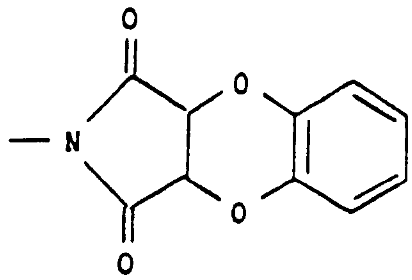
,

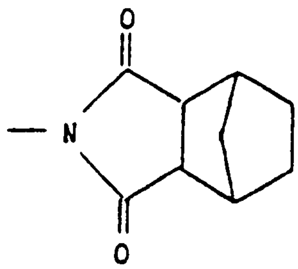
,
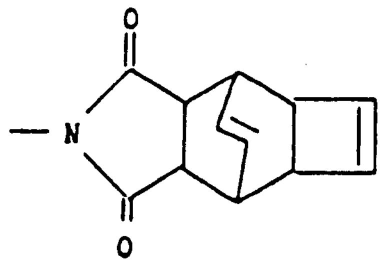

worin

- . Y und Y', die gleichartig oder verschieden sein können, ein Wasserstoffatom, ein Halogenatom, eine Alkylgruppe, eine Alkoxygruppe oder eine Hydroxylgruppe,
- . m eine ganze Zahl mit einem Wert von 1 oder 2 darstellen,
- . und das Stickstoffatom des Pyridinrings in der $\alpha$-, $\beta$-, $\gamma$- oder $\delta$-Stellung der Bindung des Rings steht, darstellen, bedeuten,

von deren Enantiomeren, Diastereoisomeren und Epimeren sowie von deren Additionssalzen mit einer pharmazeutisch annehmbaren Säure,
**dadurch gekennzeichnet**, daß man als Ausgangsmaterial ein Derivat der Formel (III):

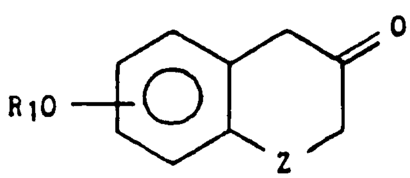
(III)

In der $R_1$ und Z die bezüglich der Formel (I) angegebenen Bedeutungen besitzen, verwendet,
welches man vorzugsweise bei Umgebungstemperatur und Umgebungsdruck in Gegenwart eines Katalysators mit einem Derivat der allgemeinen Formel (IV):

$NH_2 - (CH_2)_n - R_3$ (IV)

in der n und $R_3$ die bezüglich der Formel (I) angegebenen Bedeutungen besitzen,
einer reduktiven Aminierung unterwirft zur Bildung eines Derivats der Formel (V):

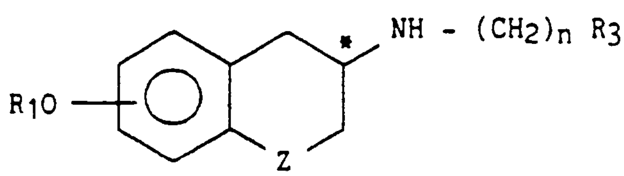
(V)

in der $R_1$, $R_3$, Z und n die oben angegebenen Bedeutungen besitzen,
einem Sonderfall der Derivate der Formel (I), worin $R_2$ ein Wasserstoffatom bedeutet,

welches man in die Isomeren (+) und (-) auftrennt, indem man eine organische Lösung bildet, dieser eine optisch aktive Säure zusetzt und stehenläßt, so daß eines der beiden Enantiomeren kristallisiert und das andere in Lösung verbleibt, wonach man das ausgewählte Isomer, wenn in der herzustellenden Verbindung der Formel (I) $R_2$ eine geradkettige oder verzweigte $C_1$-$C_6$-Alkylgruppe darstellt, mit einem Derivat der Formel (VI):

$R'_2$ - X (VI)

in der X ein Halogenatom und $R'_2$ eine geradkettige oder verzweigte $C_1$-$C_6$-Alkylgruppe bedeuten, behandelt,
so daß man spezifisch ein Enantiomer des Derivats der Formel (I) erhält.

2. Verfahren zur Herstellung von Enantiomeren der Derivate der Formel (I) nach Anspruch 1, worin $R_2$ kein Wasserstoffatom darstellt, **dadurch gekennzeichnet**, daß man ein Derivat der Formel (V) nach Anspruch 1 mit einem Derivat der Formel (VI) nach Anspruch 1 umsetzt zur Bildung des Racemats einer Verbindung der Formel (I) nach Anspruch 1, das man in die Enantiomeren auftrennt, indem man eine organische Lösung bildet, dieser eine optisch aktive Säure zugibt und durch Stehenlassen zur Kristallisation eines der beiden Enantiomere kristallisiert, während das andere in der Lösung verbleibt.

3. Verfahren zur Herstellung von Enantiomeren der Verbindungen der Formel (I) nach Anspruch 1 oder 2, **dadurch gekennzeichnet**, daß die zur Trennung der Enantiomeren verwendete Säure in Abhängigkeit von dem gewünschten Enantiomer des Produkts aus der (+)- oder (-)-Form der Citronensäure, Weinsäure, Diacetylweinsäure, Maleinsäure, Äpfelsäure, Tosylglutaminsäure, Camphersulfonsäure, Camphersäure, Camphansäure, Mandelsäure und Chinasäure ausgewählt ist.

4. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Äpfelsäure ist.

5. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Camphansäure ist.

6. Verfahren nach Anspruch 1 oder 2 zur Herstellung des Derivats der Formel (I), nämlich (+)-3-[4-[N-(5-Methoxy-chroman-3-yl)-amino]-butyl]-2,4-dioxo-3-azaspiro[4,5]decan und dessen Additionssalzen mit einer pharmazeutisch annehmbaren Säure.

7. Verfahren nach Anspruch 1 oder 2 zur Herstellung des Derivats der Formel (I), nämlich (-)-3-[4-[n-(5-Methoxy-chroman-3-yl)-amino]-butyl]-2,4-dioxo-3-azaspiro[4,5]decan und dessen Additionssalzen mit einer pharmazeutisch annehmbaren Säure.

8. Verfahren nach Anspruch 1 oder 2 zur Herstellung des Derivats der Formel (I), nämlich (+)-3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

9. Verfahren nach Anspruch 1 oder 2 zur Herstellung des Derivats der Formel (I), nämlich (-)-3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

10. Verfahren nach Anspruch 1 oder 2 zur Herstellung des Derivats der Formel (I), nämlich (+)-5-Methoxy-3-[N-n-propyl-N-[4-(4-toluol-sulfonylamino)-butyl]amino]-chroman und dessen Additionssalzen mit einer pharmazeutisch annehmbaren Säure.

11. Verfahren nach Anspruch 1 oder 2 zur Herstellung des Derivats der Formel (I), nämlich (-)-5-Methoxy-3-[N-n-propyl-N-[4-(4-toluol-sulfonylamino)-butyl]amino]-chroman und dessen Additionssalzen mit einer pharmazeutisch annehmbaren Säure.

**12.** Verfahren nach Anspruch 1 oder 2 zur Herstellung des Derivats der Formel (I), nämlich (+)-5-Methoxy-3-[N-n-propyl-N-[2-(4-toluol-sulfonylamino)-ethyl]amino]-chroman und dessen Additionssalzen mit einer pharmazeutisch annehmbaren Säure.

**13.** Verfahren nach Anspruch 1 oder 2 zur Herstellung des Derivats der Formel (I), nämlich (-)-5-Methoxy-3-[N-n-propyl-N-[2-[4-toluol-sulfonylamino)-ethyl]amino]-chroman und dessen Additionssalzen mit einer pharmazeutisch annehmbaren Säure.

**14.** Verfahren nach Anspruch 1 oder 2 zur Herstellung des Derivats der Formel (I), nämlich (+)-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

**15.** Verfahren nach Anspruch 1 oder 2 zur Herstellung des Derivats der Formel (I), nämlich (-)(-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

**16.** Additionssalze der Verbindungen der Formel (I) und von deren Enantiomeren nach Anspruch 1 mit (+)- oder (-)-Citronensäure. -Weinsäure. -Diacetylweinsäure, -Maleinsäure, -Äpfelsäure, -Tosylglutaminsäure, -Camphersulfonsäure, - Camphersäure, -Camphansäure, -Mandelsäure oder -Chinasäure.

**17.** Additionssalze der Verbindungen der Formel (I) und von ihren Enantiomeren nach Anspruch 1 mit (+)- oder (-)-Äpfelsäure.

**18.** Additionssalze der Verbindungen der Formel (I) und von ihren Enantiomeren nach Anspruch 1 mit (+)- oder (-)-Camphansäure.

**Patentansprüche für folgenden Vertragsstaat : ES**

**1.** Verfahren zur Herstellung von Enantiomeren von Derivaten der allgemeinen Formel (I):

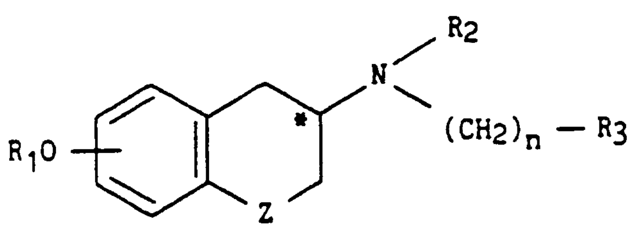

(I)

in der

- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl mit einem Wert von 1 bis 6,
- $R_3$ eine Nitrilgruppe, eine Aminogruppe, die gegebenenfalls durch eine
  . geradkettige oder verzweigte Acylgruppe mit 2 bis 7 Kohlenstoffatomen,
  . eine Alkylsulfonylgruppe,
  . eine Arylsulfonylgruppe, die gegebenenfalls durch eine Alkylgruppe, eine Alkoxygruppe, eine Hydroxylgruppe oder ein Halogenatom substituiert ist, oder
  . eine geradkettige oder verzweigte ($C_1$-$C_6$)-Alkylgruppe substituiert ist,

- oder $R_3$ eine der folgenden Gruppen:

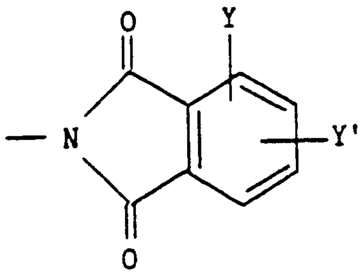

,

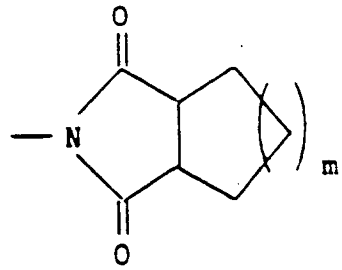

,

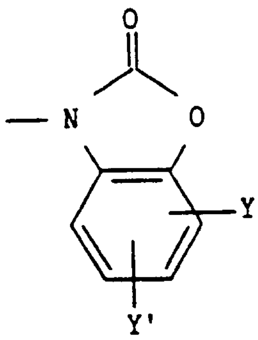

,

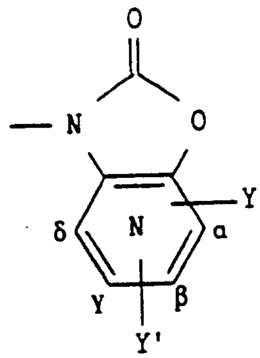

,

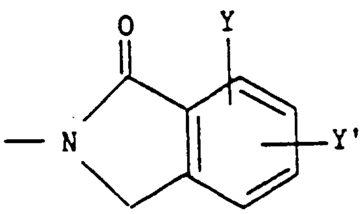

,

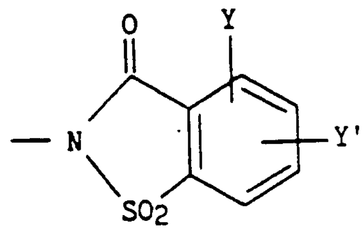

,

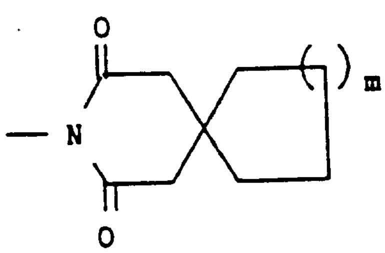

,

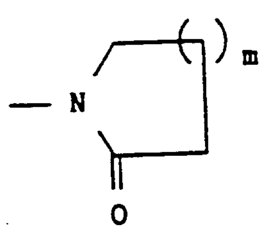

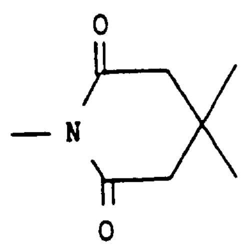

,

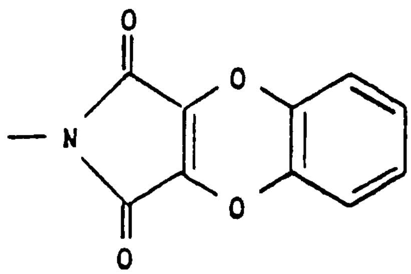

,

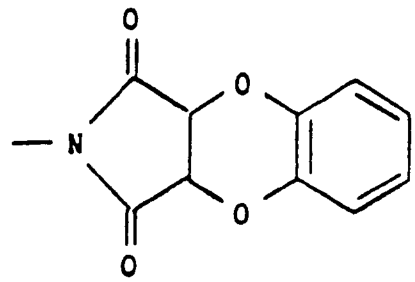

,

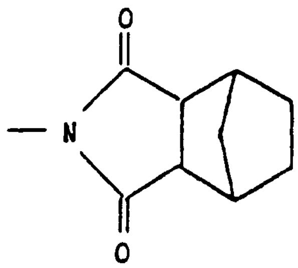

,

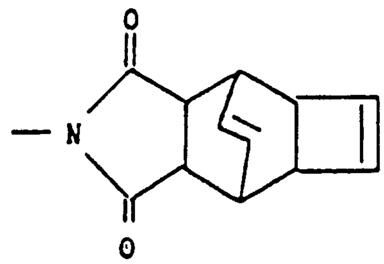

worin

. Y und Y', die gleichartig oder verschieden sein können, ein Wasserstoffatom, ein Halogenatom, eine Alkylgruppe, eine Alkoxygruppe oder eine Hydroxylgruppe,

. m eine ganze Zahl mit einem Wert von 1 oder 2 darstellen,

. und das Stickstoffatom des Pyridinrings in der $\alpha$-, $\beta$-, $\gamma$- oder $\delta$-Stellung der Bindung des Rings steht, darstellen, bedeuten,

von deren Enantiomeren, Diastereoisomeren und Epimeren sowie von deren Additionssalzen mit einer pharmazeutisch annehmbaren Säure,

**dadurch gekennzeichnet**, daß man als Ausgangsmaterial ein Derivat der Formel (III):

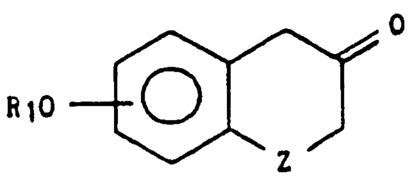

(III)

In der $R_1$ und Z die bezüglich der Formel (I) angegebenen Bedeutungen besitzen, verwendet,
welches man vorzugsweise bei Umgebungstemperatur und Umgebungsdruck in Gegenwart eines Katalysators mit einem Derivat der allgemeinen Formel (IV):

$NH_2$ - $(CH_2)_n$- $R_3$ (IV)

in der n und $R_3$ die bezüglich der Formel (I) angegebenen Bedeutungen besitzen,
einer reduktiven Aminierung unterwirft zur Bildung eines Derivats der Formel (V):

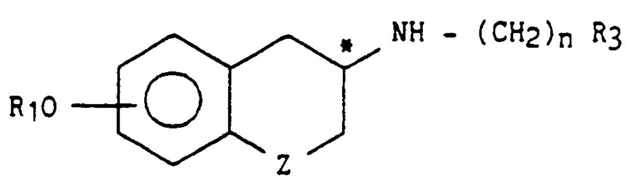

(V)

in der $R_1$, $R_3$, Z und n die oben angegebenen Bedeutungen besitzen,
einem Sonderfall der Derivate der Formel (I), worin $R_2$ ein Wasserstoffatom bedeutet,
welches man In die Isomeren (+) und (-) auftrennt, indem man eine organische Lösung bildet, dieser

eine optisch aktive Säure zusetzt und stehenläßt, so daß eines der beiden Enantiomeren kristallisiert und das andere in Lösung verbleibt, wonach man das ausgewählte Isomer, wenn in der herzustellenden Verbindung der Formel (I) $R_2$ eine geradkettige oder verzweigte $C_1$-$C_6$-Alkylgruppe darstellt, mit einem Derivat der Formel (VI):

$R'_2$ - X (VI)

in der X ein Halogenatom und $R'_2$ eine geradkettige oder verzweigte $C_1$-$C_6$-Alkylgruppe bedeuten, behandelt,
so daß man spezifisch ein Enantiomer des Derivats der Formel (I) erhält.

2. Verfahren zur Herstellung von Enantiomeren der Derivate der Formel (I) nach Anspruch 1, worin $R_2$ kein Wasserstoffatom darstellt, **dadurch gekennzeichnet**, daß man ein Derivat der Formel (V) nach Anspruch 1 mit einem Derivat der Formel (VI) nach Anspruch 1 umsetzt zur Bildung des Racemats einer Verbindung der Formel (I) nach Anspruch 1, das man in die Enantiomeren auftrennt, indem man eine organische Lösung bildet, dieser eine optisch aktive Säure zugibt und durch Stehenlassen zur Kristallisation eines der beiden Enantiomere kristallisiert, während das andere in der Lösung verbleibt.

3. Verfahren zur Herstellung von Enantiomeren der Verbindungen der Formel (I) nach Anspruch 1 oder 2, **dadurch gekennzeichnet**, daß die zur Trennung der Enantiomeren verwendete Säure in Abhängigkeit von dem gewünschten Enantiomer des Produkts aus der (+)- oder (-)-Form der Citronensäure, Weinsäure, Diacetylweinsäure, Maleinsäure, Äpfelsäure, Tosylglutaminsäure, Camphersulfonsäure, Camphersäure, Camphansäure, Mandelsäure und Chinasäure ausgewählt ist.

4. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Äpfelsäure ist.

5. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Camphansäure ist.

6. Verfahren nach Anspruch 1 oder 2 zur Herstellung des Derivats der Formel (I), nämlich (+)-3-[4-[N-(5-Methoxy-chroman-3-yl)-amino]-butyl]-2,4-dioxo-3-azaspiro[4,5]decan und dessen Additionssalzen mit einer pharmazeutisch annehmbaren Säure.

7. Verfahren nach Anspruch 1 oder 2 zur Herstellung des Derivats der Formel (I), nämlich (-)-3-[4-[n-(5-Methoxy-chroman-3-yl)-amino]-butyl]-2,4-dioxo-3-azaspiro[4,5]decan und dessen Additionssalzen mit einer pharmazeutisch annehmbaren Säure.

8. Verfahren nach Anspruch 1 oder 2 zur Herstellung des Derivats der Formel (I), nämlich (+)-3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

9. Verfahren nach Anspruch 1 oder 2 zur Herstellung des Derivats der Formel (I), nämlich (-)3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

10. Verfahren nach Anspruch 1 oder 2 zur Herstellung des Derivats der Formel (I), nämlich (+)-5-Methoxy-3-[N-n-propyl-N-[4-(4-toluol-sulfonylamino)-butyl]amino]-chroman und dessen Additionssalzen mit einer pharmazeutisch annehmbaren Säure.

11. Verfahren nach Anspruch 1 oder 2 zur Herstellung des Derivats der Formel (I), nämlich (-)-5-Methoxy-3-[N-n-propyl-N-[4-(4-toluol-sulfonylamino)-butyl]amino]-chroman und dessen Additionssalzen mit einer pharmazeutisch annehmbaren Säure.

12. Verfahren nach Anspruch 1 oder 2 zur Herstellung des Derivats der Formel (I), nämlich (+)-5-Methoxy-3-[N-n-propyl-N-[2-(4-toluol-sulfonylamino)-ethyl]amino]-chroman und dessen Additionssalzen mit einer

pharmazeutisch annehmbaren Säure.

**13.** Verfahren nach Anspruch 1 oder 2 zur Herstellung des Derivats der Formel (I), nämlich (-)-5-Methoxy-3-[N-n-propyl-N-[2-(4-toluol-sulfonylamino)-ethyl]amino]-chroman und dessen Additionssalzen mit einer pharmazeutisch annehmbaren Säure.

**14.** Verfahren nach Anspruch 1 oder 2 zur Herstellung des Derivats der Formel (I), nämlich (+)-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

**15.** Verfahren nach Anspruch 1 oder 2 zur Herstellung des Derivats der Formel (I), nämlich (-)(-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

**16.** Additionssalze der Verbindungen der Formel (I) und von deren Enantiomeren nach Anspruch 1 mit (+)- oder (-)-Citronensäure, -Weinsäure, -Diacetylweinsäure, -Maleinsäure, -Äpfelsäure, -Tosylglutaminsäure, -Camphersulfonsäure, - Camphersäure, -Camphansäure, -Mandelsäure oder -Chinasäure.

**17.** Additionssalze der Verbindungen der Formel (I) und von ihren Enantiomeren nach Anspruch 1 mit (+)- oder (-)-Äpfelsäure.

**18.** Additionssalze der Verbindungen der Formel (I) und von ihren Enantiomeren nach Anspruch 1 mit (+)- oder (-)-Camphansäure.

**Patentansprüche für folgenden Vertragsstaat : GR**

**1.** Verfahren zur Herstellung von Enantiomeren von Derivaten der allgemeinen Formel (I):

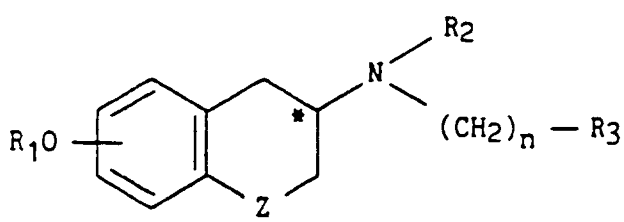

(I)

in der

- Z ein Sauerstoffatom oder ein Schwefelatom,
- $R_1$ ein Wasserstoffatom oder eine geradkettige oder verzweigte ($C_1$-$C_6$)-Alkylgruppe,
- $R_2$ ein Wasserstoffatom oder eine geradkettige oder verzweigte ($C_1$-$C_6$)-Alkylgruppe,
- n eine ganze Zahl mit einem Wert von 1 bis 6,
- $R_3$ eine Nitrilgruppe, eine Aminogruppe, die gegebenenfalls durch eine
  - . geradkettige oder verzweigte Acylgruppe mit 2 bis 7 Kohlenstoffatomen,
  - . eine Alkylsulfonylgruppe,
  - . eine Arylsulfonylgruppe, die gegebenenfalls durch eine Alkylgruppe, eine Alkoxygruppe, eine Hydroxylgruppe oder ein Halogenatom substituiert ist, oder
  - . eine geradkettige oder verzweigte ($C_1$-$C_6$)-Alkylgruppe substituiert ist,

- oder $R_3$ eine der folgenden Gruppen:

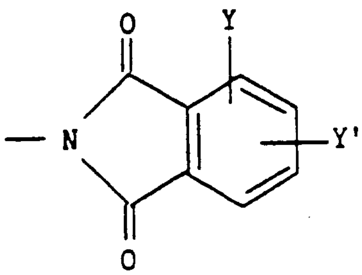
,
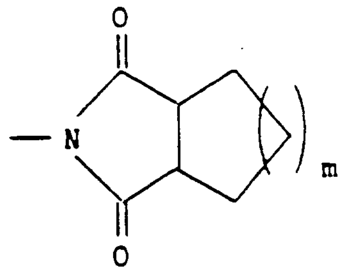
,
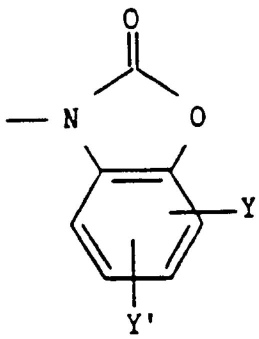
,
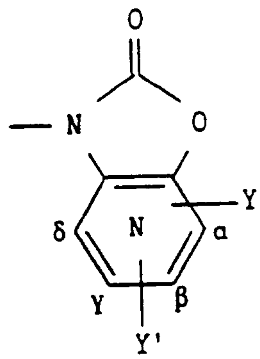
,
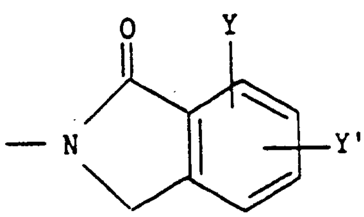
,
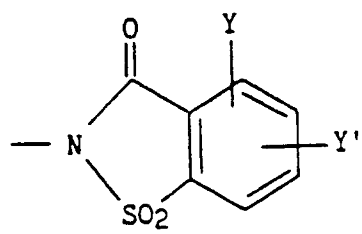
,
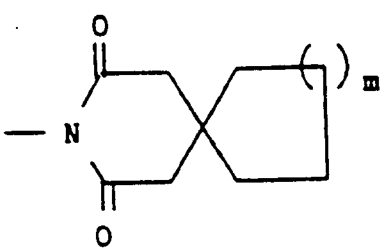
,
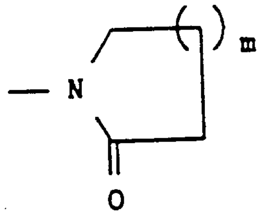
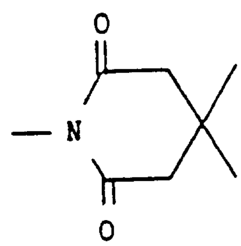
,

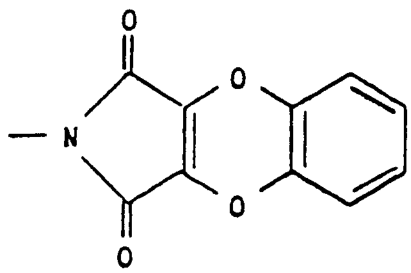 , 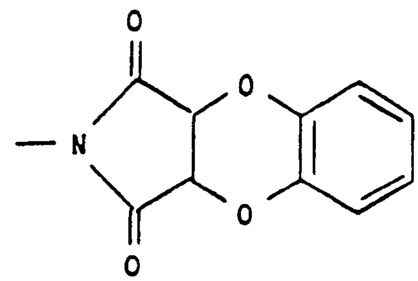 ,

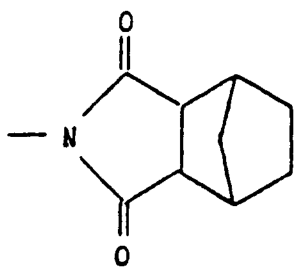 , 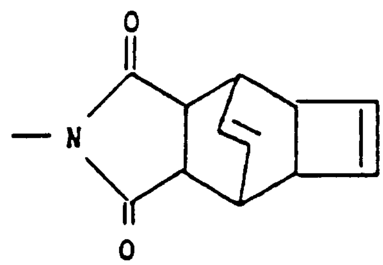

worin

- . Y und Y', die gleichartig oder verschieden sein können, ein Wasserstoffatom, ein Halogenatom, eine Alkylgruppe, eine Alkoxygruppe oder eine Hydroxylgruppe,
- . m eine ganze Zahl mit einem Wert von 1 oder 2 darstellen,
- . und das Stickstoffatom des Pyridinrings in der α-, β-, γ- oder δ-Stellung der Bindung des Rings steht, darstellen, bedeuten,

von deren Enantiomeren, Diastereoisomeren und Epimeren sowie von deren Additionssalzen mit einer pharmazeutisch annehmbaren Säure,

**dadurch gekennzeichnet**, daß man als Ausgangsmaterial ein Derivat der Formel (III):

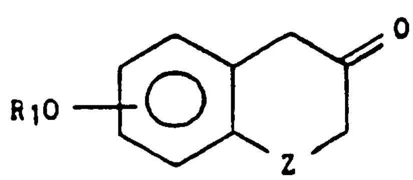

(III)

in der $R_1$ und Z die bezüglich der Formel (I) angegebenen Bedeutungen besitzen, verwendet,
welches man vorzugsweise bei Umgebungstemperatur und Umgebungsdruck in Gegenwart eines Katalysators mit einem Derivat der allgemeinen Formel (IV):

$NH_2$ - $(CH_2)_n$- $R_3$ (IV)

in der n und $R_3$ die bezüglich der Formel (I) angegebenen Bedeutungen besitzen,
einer reduktiven Aminierung unterwirft zur Bildung eines Derivats der Formel (V):

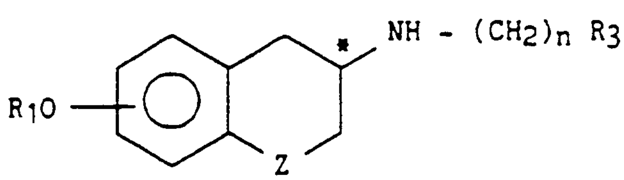

(V)

in der $R_1$, $R_3$, Z und n die oben angegebenen Bedeutungen besitzen,
einem Sonderfall der Derivate der Formel (I), worin $R_2$ ein Wasserstoffatom bedeutet,
welches man in die Isomeren (+) und (-) auftrennt, indem man eine organische Lösung bildet, dieser

eine optisch aktive Säure zusetzt und stehenläßt, so daß eines der beiden Enantiomeren kristallisiert und das andere in Lösung verbleibt, wonach man das ausgewählte Isomer, wenn in der herzustellenden Verbindung der Formel (I) $R_2$ eine geradkettige oder verzweigte $C_1$-$C_6$-Alkylgruppe darstellt, mit einem Derivat der Formel (VI):

$R'_2$ - X (VI)

in der X ein Halogenatom und $R'_2$ eine geradkettige oder verzweigte $C_1$-$C_6$-Alkylgruppe bedeuten, behandelt,
so daß man spezifisch ein Enantiomer des Derivats der Formel (I) erhält.

2. Verfahren zur Herstellung von Enantiomeren der Derivate der Formel (I) nach Anspruch 1, worin $R_2$ kein Wasserstoffatom darstellt, **dadurch gekennzeichnet**, daß man ein Derivat der Formel (V) nach Anspruch 1 mit einem Derivat der Formel (VI) nach Anspruch 1 umsetzt zur Bildung des Racemats einer Verbindung der Formel (I) nach Anspruch 1, das man in die Enantiomeren auftrennt, indem man eine organische Lösung bildet, dieser eine optisch aktive Säure zugibt und durch Stehenlassen zur Kristallisation eines der beiden Enantiomere kristallisiert, während das andere in der Lösung verbleibt.

3. Verfahren zur Herstellung von Enantiomeren der Verbindungen der Formel (I) nach Anspruch 1 oder 2, **dadurch gekennzeichnet**, daß die zur Trennung der Enantiomeren verwendete Säure in Abhängigkeit von dem gewünschten Enantiomer des Produkts aus der (+)- oder (-)-Form der Citronensäure, Weinsäure, Diacetylweinsäure, Maleinsäure, Äpfelsäure, Tosylglutaminsäure, Camphersulfonsäure, Camphersäure, Camphansäure, Mandelsäure und Chinasäure ausgewählt ist.

4. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Äpfelsäure ist.

5. Verfahren zur Herstellung von Enantiomeren der Derivate der Formel (I) nach Anspruch 1 oder 2, **dadurch gekennzeichnet**, daß die zur Trennung der Isomeren verwendete Säure in Abhängigkeit von dem gewünschten Enantiomer (+)- oder (-)-Camphansäure ist.

6. Verfahren nach Anspruch 1 oder 2 zur Herstellung des Derivats der Formel (I), nämlich (+)-3-[4-[N-(5-Methoxy-chroman-3-yl)-amino]-butyl]-2,4-dioxo-3-azaspiro[4,5]decan und dessen Additionssalzen mit einer pharmazeutisch annehmbaren Säure.

7. Verfahren nach Anspruch 1 oder 2 zur Herstellung des Derivats der Formel (I), nämlich (-)-3-[4-[n-(5-Methoxy-chroman-3-yl)-amino]-butyl]-2,4-dioxo-3-azaspiro[4,5]decan und dessen Additionssalzen mit einer pharmazeutisch annehmbaren Säure.

8. Verfahren nach Anspruch 1 oder 2 zur Herstellung des Derivats der Formel (I), nämlich (+)-3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

9. Verfahren nach Anspruch 1 oder 2 zur Herstellung des Derivats der Formel (I), nämlich (-)-3-[4-[N-n-Propyl-N-(5-methoxy-chroman-3-yl)-amino]-butyl]-2,4-dioxo-3-azaspiro[4,5]decan und dessen Additionssalzen mit einer pharmazeutisch annehmbaren Säure.

10. Verfahren nach Anspruch 1 oder 2 zur Herstellung des Derivats der Formel (I), nämlich (+)-5-Methoxy-3-[N-n-propyl-N-[4-(4-toluol-sulfonylamino)-butyl]amino]-chroman und dessen Additionssalzen mit einer pharmazeutisch annehmbaren Säure.

11. Verfahren nach Anspruch 1 oder 2 zur Herstellung des Derivats der Formel (I), nämlich (-)-5-Methoxy-3-[N-n-propyl-N-[4-(4-toluol-sulfonylamino)-butyl]amino]-chroman und dessen Additionssalzen mit einer pharmazeutisch annehmbaren Säure.

12. Verfahren nach Anspruch 1 oder 2 zur Herstellung des Derivats der Formel (I), nämlich (+)-5-Methoxy-3-[N-n-propyl-N-[2-(4-toluol-sulfonylamino)-ethyl]amino]-chroman und dessen Additionssalzen mit einer

pharmazeutisch annehmbaren Säure.

13. Verfahren nach Anspruch 1 oder 2 zur Herstellung des Derivats der Formel (I), nämlich (-)-5-Methoxy-3-[N-n-propyl-N-[2-(4-toluol-sulfonylamino)-ethyl]amino]-chroman und dessen Additionssalzen mit einer pharmazeutisch annehmbaren Säure.

14. Verfahren nach Anspruch 1 oder 2 zur Herstellung des Derivats der Formel (I), nämlich (+)-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

15. Verfahren nach Anspruch 1 oder 2 zur Herstellung des Derivats der Formel (I), nämlich (-)(-3-{4-[N-n-Propyl-N-(5-methoxy-chroman-3-yl)-amino]-butyl]-2,4-dioxo}-3-azabicyclo[3.3.0]octan und dessen Additionssalzen mit einer pharmazeutisch annehmbaren Säure.

16. Additionssalze der Verbindungen der Formel (I) und von deren Enantiomeren nach Anspruch 1 mit (+)- oder (-)-Citronensäure, -Weinsäure, -Diacetylweinsäure, -Maleinsäure, -Äpfelsäure, Tosylglutaminsäure, -Camphersulfonsäure, - Camphersäure, -Camphansäure, -Mandelsäure oder -Chinasäure.

17. Additionssalze der Verbindungen der Formel (I) und von ihren Enantiomeren nach Anspruch 1 mit (+)- oder (-)-Äpfelsäure.

18. Additionssalze der Verbindungen der Formel (I) und von ihren Enantiomeren nach Anspruch 1 mit (+)- oder (-)-Camphansäure.